# EUROPÄISCHE PATENTSCHRIFT

(11) **EP 0 869 184 B1**
(45) Veröffentlichungstag und Bekanntmachung des Hinweises auf die Patenterteilung: **04.10.2001**
(21) Anmeldenummer: 98107383.6
(22) Anmeldetag: 06.07.1995
(51) Int. Cl.: C12N 15/40, C07K 14/18, A61K 39/12, C12N 15/85

(54) **Vakzine zur Immunisierung gegen TBE-Virusinfektionen sowie ein Verfahren zu dessen Herstellung**
Vaccin for immunisation against TBE viral infections and method or its preparation
Vaccin pour l'immunisation contre les infections par des virus TBE et procédé pour sa préparation

(30) Priorität: 08.07.1994 AT 135294; 23.05.1995 AT 87195
(43) Veröffentlichungstag der Anmeldung: 07.10.1998
(62) Teilanmeldung aus: 95890132.4
(73) Patentinhaber: Baxter Aktiengesellschaft, 1221 Wien (AT)
(72) Erfinder: Heinz, Xaver-Franz, Prof.Dr., A-1170 Wien (AT); Allison, Steven, Dr., 1190 Wien (AT); Mandl, Christian, Doz. Dr., 1160 Wien (AT); Kunz, Christian, Prof. Dr., 1180 Wien (AT)
(74) Vertreter: Alge, Daniel, Mag. Dr. rer.nat.

(56) Entgegenhaltungen:
- EP-A- 0 284 791
- WO-A-92/03545
- EIJI KONISHI ET AL.: "Mice immunized with a subviral particle containing the Japanese Encephalitis Virus prM/M and E proteins are protected from lethal JEV infection" VIROLOGY, Bd. 188, Nr. 2, Juni 1992, Seiten 714-720, XP002026147 ORLANDO US
- CHRISTIAN W. MANDL ET AL.: "Sequence of the structural proteins of Tick-borne encephalitis virus (Western subtype) and comparative analysis with other Flaviviruses" VIROLOGY, Bd. 168, 1988, Seiten 197-205, XP000602618 ORLANDO US
- KONISHI, EIJI ET AL: "Proper maturation of the Japanese encephalitis virus envelope glycoprotein requires cosynthesis with the premembrane protein" J. VIROL. (1993), 67(3), 1672-5 CODEN: JOVIAM;ISSN: 0022-538X,1993, XP000618522
- HOLZMANN H ET AL: "MOLECULAR EPIDEMIOLOGY OF TICK - BORNE ENCEPHALITIS VIRUS CROSS-PROTECTION BETWEEN EUROPEAN AND FAR EASTERN SUBTYPES." VACCINE 10 (5). 1992. 345-349. CODEN: VACCDE ISSN: 0264-410X, XP002026148
- ALLISON, STEVEN L. ET AL: "Expression of cloned envelope protein genes from the flavivirus tick - borne encephalitis virus in mammalian cells and random mutagenesis by PCR" VIRUS GENES (1994), 8(3), 187-98 CODEN: VIGEET;ISSN: 0920-8569,1994, XP000618523
- JEFFREY B. ULMER ET AL.: "Heterologous protection against influenza by injection of DNA encoding a viral protein", SCIENCE, , 19. Maerz 1993, Band 259, Nr. , Seiten 1745 - 1749

## Beschreibung

Die Erfindung betrifft eine verbesserte Vakzine zur Immunisierung gegen TBE-Virusinfektionen sowie ein Verfahren zu dessen Herstellung.

Das TBE (Tick-Borne-Encephalitis)-Virus ist in vielen europäischen Ländern, der ehemaligen Sowjetunion und China, endemisch. Die Krankheit stellt in einigen zentraleuropäischen Ländern, wie Österreich, Tschechien, Slowakei, Slowenien oder Ungarn, wo jedes Jahr mehrere hundert in Krankenhäuser aufgenommene Fälle registriert werden, ein signifikantes Problem für die öffentliche Gesundheit dar (WHO: EURO Reports and Studies, 104; 1983).

TBE-Virus, das in Form eines westlichen, europäischen und eines fernöstlichen Subtyps existiert, gehört zur Familie der Flavi-Viren, die sphärische lipidumhüllte RNA-Viren sind (s. Monath, T.P.: Flaviviruses. In: Fields, B.N. (ed.) Virology, Raven Press, N.Y. 1990, S. 763-814).

Das Flavi-Virus-Virion im allgemeinen besteht aus einem Nukleocapsid, welches das Positivstrang-RNA-Genom in Verbindung mit dem viralen Capsid (C)-Protein enthält. Das Nukleocapsid ist von einer Lipidhülle umgeben, die die Membran-assoziierten Proteine E (50 bis 60 kD) und M (7 bis 8 kD) enthält (Heinz und Roehrig in: van Regenmortel und Neurath (Hrsgb.) "Immunochemistry of Viruses II. The Basis for Seradiagnosis and Vaccines. Elsevier Sciences, Amsterdam (1990), 289-305).

Das Haupthüllprotein E spielt eine zentrale Rolle in der Biologie von Flavi-Viren, indem es bedeutende virale Eintrittsfunktionen vermittelt und eine protektive Immunantwort im Wirt auslöst. Es gibt bereits eine beträchtliche Menge an Informationen betreffend die Struktur des Hüllproteins E des TBE-Virus und es wurde ein Strukturmodell auf Basis einer Vielzahl von biochemischen und immunologischen Daten vorgeschlagen (Mandl et al., J. Virol. 63 (1989), 564-571).

Die Krankheit kann durch Impfung mit einem hochgereinigten Formalin-inaktivierten Ganzvirusimpfstoff (s. Kunz et al., J.Med. Virol., 6 (1980), 103-109), der eine Immunantwort gegen die Strukturproteine des Virus induziert, effektiv verhindert werden (Kunz, Ch.Acta leidensia 60, No.2, 1-14, 1992). Dieser Impfstoff hat sich bestens bewährt, jedoch müssen große Volumina von infektiösen und potentiell gefährlichen Virussuspensionen im Verlauf des Herstellungsverfahrens gehandhabt werden. Daher sind umfangreiche und teure Sicherheitsvorkehrungen erforderlich.

Die Fähigkeit von Antikörpern, das Virus zu neutralisieren, hängt davon ab, wie effizient sie die native Struktur der Proteine an der Virusoberfläche erkennen. Im Falle von TBE-Viren und anderen Flavi-Viren handelt es sich dabei in erster Linie um das Protein E (s. Heinz und Mandl, APMIS, 101 (1994), 735-745). Für die Induktion von möglichst effizient wirksamen Antikörpern im Zuge einer Immunisierung ist es daher wünschenswert, daß der Impfstoff dieses Protein in der gleichen Form, wie es auch an der Oberfläche des infektiösen Virus vorliegt, enthält. Ganzvirustotimpfstoffe haben den Nachteil, daß das notwendige Inaktivierungsverfahren zu einer teilweisen Veränderung der nativen Proteinstruktur führen kann.

Die rekombinante DNA-Technologie bietet eine Möglichkeit, Ganzvirustotimpfstoffe durch rekombinante Proteine, die die wesentlichen Teile der die Immunantwort induzierenden Proteine enthalten, zu ersetzen. Bei dieser gentechnischen Expression von einzelnen Virusproteinen ist jedoch nicht sichergestellt, daß die Antigenstruktur dieser rekombinanten Proteine jener der entsprechenden Proteine an der Virusoberfläche entspricht.

Die Expression von rekombinanten Oberflächenproteinen von TBE-Viren ist beispielsweise aus Allison et al., Gemeinsame Jahrestagung ÖBG-ÖGGGT (1993) P114, bekannt. Dabei wurde festgestellt, daß Protein E und Protein M bei deren rekombinanter Expression unter bestimmten Bedingungen in verschiedener Form, u.a. auch in Form von nicht-infektiösen subviralen Partikeln, abgesondert werden.

Solche subvirale Partikel sind für entfernte Verwandte der TBE-Viren, nämlich das Japanische Enzephalitis-Virus (JEV), Gelbfiebervirus und Dengue-Virus bekannt (s. Konishi et al., Virology 188 (1992), 714-720, oder WO 92/03545).

Es ist zwar in Konishi et al. beschrieben, daß solche subviralen Partikel, welche mit Freund's komplettem Adjuvans emulsifiziert worden sind und das gesamte Protein E enthalten, eine gewisse Immunantwort in Mäusen auslösen, es wurde jedoch andererseits nachgewiesen, daß mit einem teilweise C-terminal verkürzten Protein E ein wesentlich effektiverer Schutz als mit dem gesamten Protein E gegen eine Flavivirusinfektion erreicht werden kann (WO 92/03161).

In der EP 0 284 791 A werden DNA- und RNA-Sequenzen zur Herstellung eines Lebendimpfstoffes offenbart, wobei der Lebendimpfstoff ein rekombinantes Vaccinia-Virus ist, das die für TBEV-Proteine kodierende DNA enthält. Es wird also ein rekombinantes Virus, das als Lebendimpfstoff eingesetzt wird, beschrieben. Dieser Lebendimpfstoff wird jedoch nicht als "nackte Nukleinsäure" direkt zur Immunisierung eingesetzt.

Die Aufgabe der vorliegenden Erfindung besteht nun darin, einen verbesserten Impfstoff gegen Flavi-Virus-Infektionen, insbesondere TBE-Infektionen, zur Verfügung zu stellen.

Diese Aufgabe wird erfindungsgemäß gelöst durch eine Nukleinsäure-Vakzine zur Immunisierung gegen Flavi-Virus-Infektionen, enthaltend eine Nukleinsäure, welche die für Protein E und Protein prM/M kodierende Nukleinsäuresequenz, abgeleitet aus einem Flavi-Virus, in deren vollständiger nativer Form.

Es wurde überraschenderweise gefunden, daß die Nukleinsäure, welche die erwähnten Kodierungssequenzen umfaßt, als solche zur Immunisierung gegen TBE-Virusinfektionen verwendet werden kann.

Es ist im Stand der Technik bekannt, daß das Einbringen von "nackter" DNA in Mäusen eine Immunantwort auslösen kann. Beispielsweise können Mäuse, denen ein Plasmid, enthaltend eine genomische Kopie des menschlichen Wachstumshormons (hGH) injiziert wurde, Antikörper gegen menschliches hGH entwickeln (Nature, 356 (1992), 152-154).

Weiters sind einige erfolgreiche "genetische Immunisierungen" durch nackte DNA beschrieben worden. Bei dieser genetischen Immunisierung wurde DNA, welche für ein oder mehrere Antigene eines Virus kodiert, verabreicht, worauf in vivo die entsprechenden viralen Antigene synthetisiert worden sind, welche separat jedes für sich eine Immunantwort auslösen und damit in weiterer Folge einen Schutz vor Virusinfektionen bewirken kann. Erfolgreiche protektive Immunität in Mäusen durch intramuskuläre Injektion von Influenza-Virus-DNA wurde in PNAS 91 (1994), Seiten 9519-9523 (Raz et al.) beschrieben. Eine ebenfalls erfolgreiche Immunisierung von Ratten und Mäusen gegen das Hepatitis B-Virus (HBV)-Oberflächenantigen durch intramuskuläre Injektion von Plasmid-DNA, welche für das HBV-Oberflächenantigen kodierende Sequenzen enthält, wurde in Vaccine 12 (16), (1994), Seiten 1503-1509 (Davis et al.) beschrieben. Viele Versuche zur Immunisierung mit nackter DNA kodierend für Pathogen-Antigene blieben aber erfolglos. Beispielsweise wurden Versuche zur Immunisierung gegen HIV mittels direkten DNA-Transfer in Körperzellen zwar beschrieben (WO 93/17706), eine erfolgreiche Vakzine konnte bisher mit dieser Methodik jedoch nicht erhalten werden. Insbesondere scheint es, daß es für eine erfolgreiche immunisierende Wirkung der reinen DNA-Vakzine - neben dem Einbringen der DNA in die Zelle - besonders vorteilhaft ist, daß das die Immunantwort auslösende Antigen in der nativen Form dem Immunsystem präsentiert wird. Die exakte Struktur der nativen Form bzw. die biosynthetischen Vorgänge, die zur Bildung der nativen Form und Struktur erforderlich sind, sind nur für wenige Pathogene gesichert, so daß eine effektive Immunisierung mit nackter Nukleinsäure sich in vielen Fällen äußerst schwierig gestaltet, wenn nicht - aufgrund von mangelnden genauen Kenntnissen der Antigenstrukturgar unrealisierbar ist mit dem gegenwärtigen Wissensstand.

Im Rahmen der vorliegenden Erfindung hat sich herausgestellt, daß eine Immunisierung mit einer Nukleinsäuresequenz, welche ausschließlich für das die wesentliche Immunantwort bei TBE-Infektionen auslösende Protein E kodiert, nicht ausreicht, um eine Immunantwort zu erhalten, die vor einer Erkrankung schützt.

Überraschenderweise konnte eine erfolgreiche Immunantwort nur durch Verabreichung von Nukleinsäuresequenzen erhalten werden, welche neben der Kodierungssequenz für das Protein E in seiner vollständigen, nativen Form noch Kodierungssequenzen für das Protein prM/M umfaßt. Es zeigte sich weiters, daß mit einer Nukleinsäure, enthaltend eine Protein E-Kodierungssequenz, welche eine Deletion des Ankerbereiches des Protein E aufweist, ebenfalls keine erfolgreiche Immunisierung erhalten werden konnte.

Die vorliegende Erfindung betrifft gemäß einem weiteren Aspekt eine Vakzine zur Immunisierung gegen Tick-Borne-Enzephalitis-Virus (TBE-Virus)-Infektionen, umfassend eine Nukleinsäure, welche für Protein E und Protein prM/M, abgeleitet aus dem TBE-Virus, in deren zumindest im wesentlichen vollständiger, nativen Form kodiert.

Mit der vorliegenden Erfindung konnte erstmals eine Nukleinsäureimmunisierung für Flavi-Viren im allgemeinen gezeigt werden. Das erfindungsgemäße Immunisierungssystem ist daher prinzipiell nicht nur auf TBE-Virusimmunisierungen, sondern - wegen der großen Homologie aller Flavi-Viren in Bezug auf das Protein E und prM/M (s. Chambers et al., Annual Review of Microbiology, Vol. 44, 1990, S. 649-688: Flavivirus Genome Organization, Expression and Replication) - allgemein auf Immunisierungen gegen Flavi-Virusinfektionen anwendbar.

Wesentlich für eine Nukleinsäure-Vakzine zur Immunisierung gegen TBE-Virus ist, daß Protein E in seiner im wesentlichen vollständigen nativen Form von der Nukleinsäure kodiert wird. Selbstverständlich fallen Vakzine mit Nukleinsäuren, welche sich aus der Degeneration des genetischen Kodes aus dem Austausch von nichtstrukturwesentlichen Aminosäurekodons oder aus natürlichen oder im Labor erzeugten Mutationen ergeben, ebenfalls unter die vorliegende Erfindung, solange mit diesen Nukleinsäuremodifikationen der Kodierungssequenz des Proteins E eine erfolgreiche Immunantwort hervorgerufen werden kann. Vakzine enthaltend Nukleinsäuren mit Deletionen oder Insertionen in der Kodierungssequenz des Proteins E, welche die für die Immunisierung erforderliche Struktur des Proteins E im antigenen Epitopbereich im wesentlichen unverändert lassen, sind ebenfalls als in den Rahmen der Erfindung fallend anzusehen, da sie als von der TBE-Virussequenz abgeleitet gelten. Entsprechendes gilt selbstverständlich auch für die Kodierungssequenz des Proteins prM/M. Da das Protein prM/M bedeutend ist für eine zuverlässige Bildung und Sekretion der subviralen Partikel, sind Sequenzabweichungen bezüglich des Proteins prM/M von nicht so entscheidender Bedeutung wie bei Protein E, solange das erfolgreiche Assembling des Partikels gewährleistet ist. Die Natur der Nukleinsäure ist nicht erfindungswesentlich. RNA und DNA können gleichermaßen verwendet werden, wobei DNA wegen der erhöhten Stabilität gegenüber RNA in manchen Anwendungsbereichen Vorteile bietet. Die Nukleinsäure kann gleichermaßen biologisch oder durch chemische Synthese hergestellt werden.

Vorzugsweise ist die für Protein E kodierende Nukleinsäure aus dem europäischen oder fernöstlichen Subtyp des TBE-Virus abgeleitet, da dies besonders verbreitete Subtypen sind.

Besonders bevorzugte Nukleinsäure-Vakzine sind solche, bei denen die Nukleinsäure ein Plasmidvektor ist. Als besonders geeignete Plasmidvektoren haben sich solche herausgestellt, welche starke Promotoren, wie z.B. den HSV-, RSV-, EBV-, β-Actin-, Adeno-, MMTV-, hsp- und hGH-Promotor enthalten. Starke Promotoren ermöglichen eine effiziente Genexpression.

Zu den geeignetsten Vektoren bzw. Promotoren zählen die vom Plasmid pCMVβ (Mac Gregor et al.) abgeleiteten Plasmide, welche den CMV-"Immediate Early"-Promotor enthalten.

Selbstverständlich können die Nukleinsäuren der erfindungsgemäßen Nukleinsäure-Vakzine noch weitere kodierende oder nichtkodierende Sequenzen umfassen, insbesondere wenn die Nukleinsäure als Nukleinsäurevektor zur Verfügung gestellt wird.

Beispiele für solche weiteren Sequenzen sind - neben den Promotoren - noch Markergene, weitere Regulatoren betreffend Transkription, Translation oder Posttranslations-Modifikationen, etc. Bei diesen komplexeren Nukleinsäure-Konstrukten ist jedoch stets darauf zu achten, daß nach der Einbringung der Nukleinsäure in die Zielzelle durch Translation der Nukleinsäure kein infektiöses Virus gebildet werden kann, die Vakzine also ein "Totimpfstoff" ist bzw. bleibt.

Die erfindungsgemäße Nukleinsäure-Vakzine kann in der einfachsten Form die "nackte" Nukleinsäure, gegebenenfalls in einem geeigneten Puffersystem, enthalten. Weitere Bestandteile, wie arzneimitteltechnische Zusatzstoffe, Trägersubstanzen oder spezielle verabreichungsspezifische Substanzen können selbstverständlich ebenfalls vorhanden sein.

Die Applikation der erfindungsgemäßen Nukleinsäure-Vakzine erfolgt bevorzugterweise durch Injektion, insbesondere durch intramuskuläre, intradermale oder subkutane Injektion, kann aber gleichwohl auch oral erfolgen.

Die verabreichte Menge der erfindungsgemäßen Nukleinsäure-Vakzine richtet sich nach der Art der Verabreichung und dem verwendeten Adjuvans. Im Fall einer intramuskulären Administration ist die verabreichte Menge in der Regel höher als bei einer intradermalen Administration, um die gleiche protektive Immunantwort zu erhalten. Die bevorzugt verwendete Menge liegt im allgemeinen im Bereich von 0,01 ng bis 10 mg/Dosis, am meisten bevorzugt im Bereich von 1 ng bis 1 mg/Dosis.

Die erfindungsgemäße Nukleinsäure-Vakzine hat den wesentlichen Vorteil, daß keine Zellkultursysteme zur Herstellung der subviralen Partikel eingesetzt werden müssen, sondern daß die Partikel in vivo gebildet werden und so direkt eine Immunantwort auslösen können.

Die erfindungsgemäße Nukleinsäure-Vakzine hat weiterhin im Gegensatz zu Vakzinen, welche auf der Grundlage von Proteinen beruhen, den Vorteil, daß sie eine große Stabilität aufweist, insbesondere, wenn die Nukleinsäure eine DNA ist. Damit ist es möglich, Vakzine auch ohne großen Kühlungsaufwand für sehr lange Zeit zu lagern, wobei durch diese Lagerung keine wesentlichen Aktivitätsverluste zu erwarten sind. Insbesondere in lyophilisierter Form kann die Nukleinsäure für eine praktisch unbegrenzte Zeitdauer unbeschadet sogar bei Raumtemperatur gelagert werden. Auch die Rekonstitution einer lyophilisierten erfindungsgemäßen Nukleinsäure-Vakzine kann viel einfacher erfolgen als die Rekonstitution einer lyophilisierten Proteinlösung, welche erfahrungsgemäß oft auf Grund der Natur des Proteins Probleme aufwerfen kann.

Ein weiterer Vorteil der erfindungsgemäßen Nukleinsäure-Vakzine ist, daß im Gegensatz zu konventionellen Totimpfstoffsystemen, das immunisierende Antigen in vivo synthetisiert wird und zur Ausbildung einer effizienten zellulären Immunantwort führt (Science, Vol. 259, 19. März 1993, S. 1691-1692, Research News: Naked DNA Points Way to Vaccines).

Gemäß einem weiteren Aspekt betrifft die Erfindung Nukleinsäuresequenzen, insbesondere Nukleinsäurevektoren, umfassend die gesamte Kodierungssequenz der vom TBE-Virus abgeleiteten Proteine E und prM/M als Arzneimittel. Bisher konnte im Stand der Technik noch über keine einzige erfolgreiche Nukleinsäureimmunisierung mit Flavi-Virus-DNA berichtet werden. Die medizinische Verwendung dieser Nukleinsäure(-vektoren) stellt daher eine überraschende neue Verwendungsmöglichkeit dar. Unter Vektor wird erfindungsgemäß jegliches Nukleinsäurevehikel verstanden, also insbesondere Plasmide, Viren oder Transposons.

Bisher wurde lediglich ein von SV40 abgeleiteter Plasmidvektor, welcher die Proteinsequenz der von TBE-Virus abgeleiteten Proteine prM/M und E enthält, beschrieben (Allison et al., Virus-Genes 8 (3), (1994), Seiten 187-198). Für diese SV40-Plasmide wurde jedoch die Möglichkeit einer Immunisierungswirkung nicht diskutiert.

Die erfindungsgemäßen Plasmidvektoren unterscheiden sich vor allem von den bisher beschriebenen Plasmidvektoren durch ihre Eignung zur Immunisierung. Daher liegen die erfindungsgemäßen Plasmidvektoren insbesondere als verabreichungsfertige Lösungen oder Lyophilisate in einer zur Verabreichung geeigneten Spritze bzw. Ampulle vor.

Bei Plasmidvektoren haben sich erfindungsgemäß Promotoren als wirksam herausgestellt, welche aus der Gruppe CMV-, HSV-, RSV-, EBV-, β-Actin-, Adeno-, MMTV-, hsp- und hGH-Promotor ausgewählt sind, wobei sich insbesondere der CMV-"Immediate Early"-Promotor als besonders effizient erwiesen hat.

Gemäß einem weiteren Aspekt betrifft die vorliegende Erfindung die Verwendung einer Nukleinsäure, umfassend die gesamte Kodierungssequenz der vom TBE-Virus abgeleiteten Proteine prM/M und E zur Herstellung einer Vakzine.

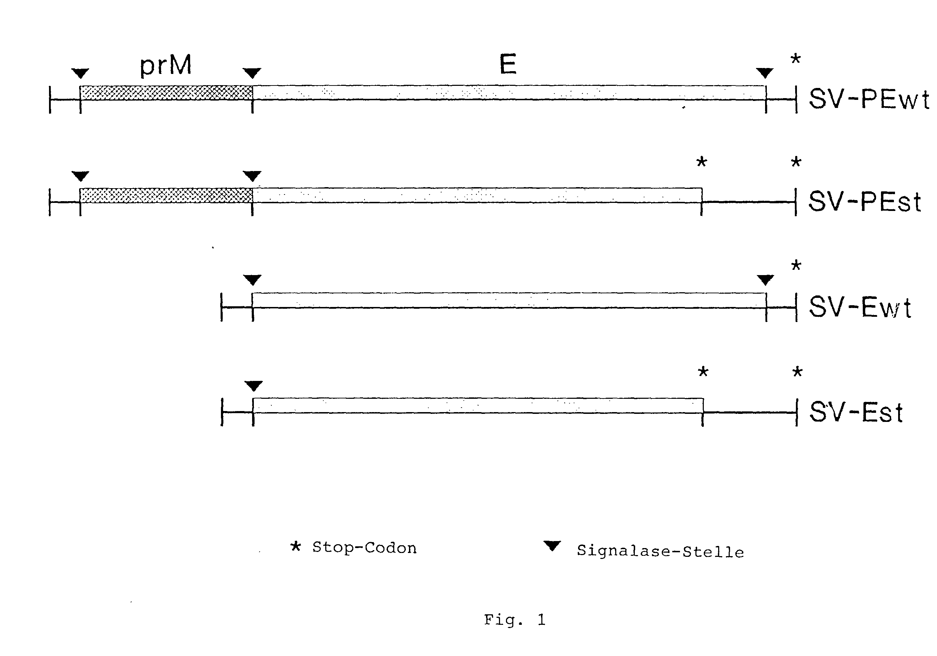

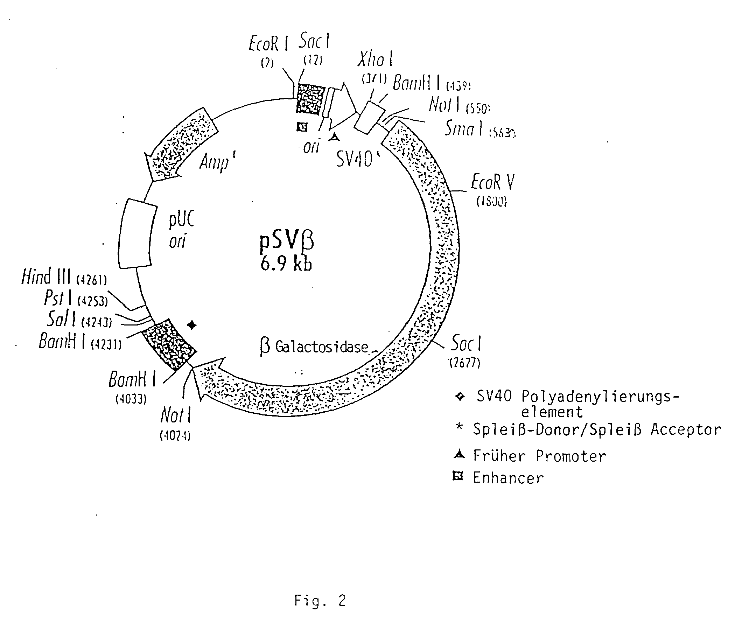

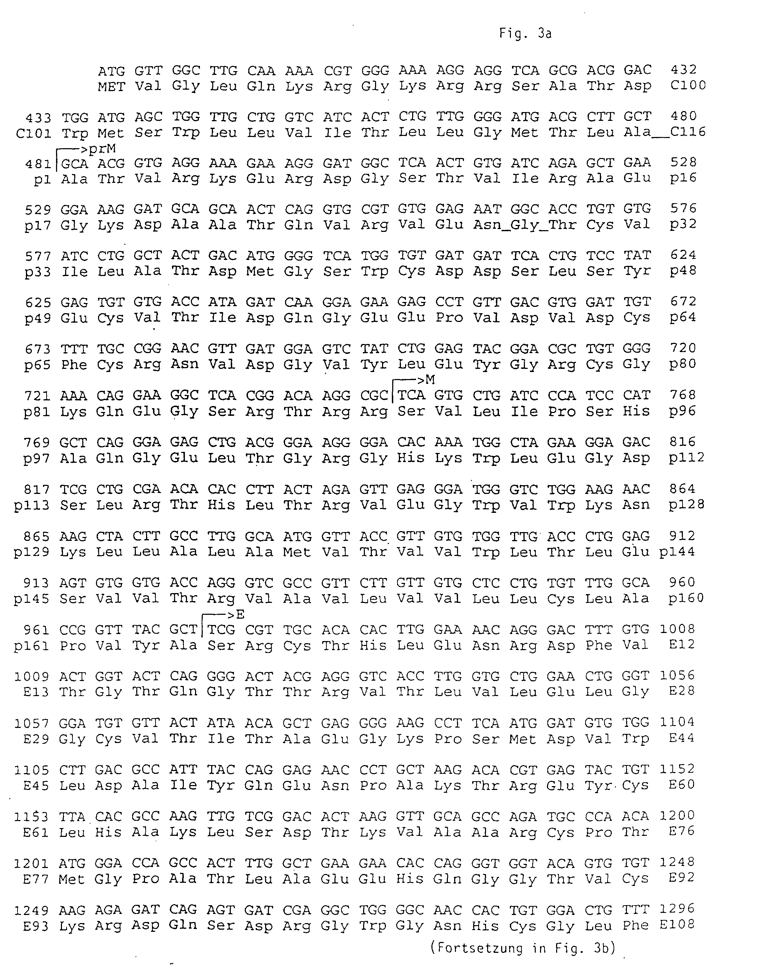

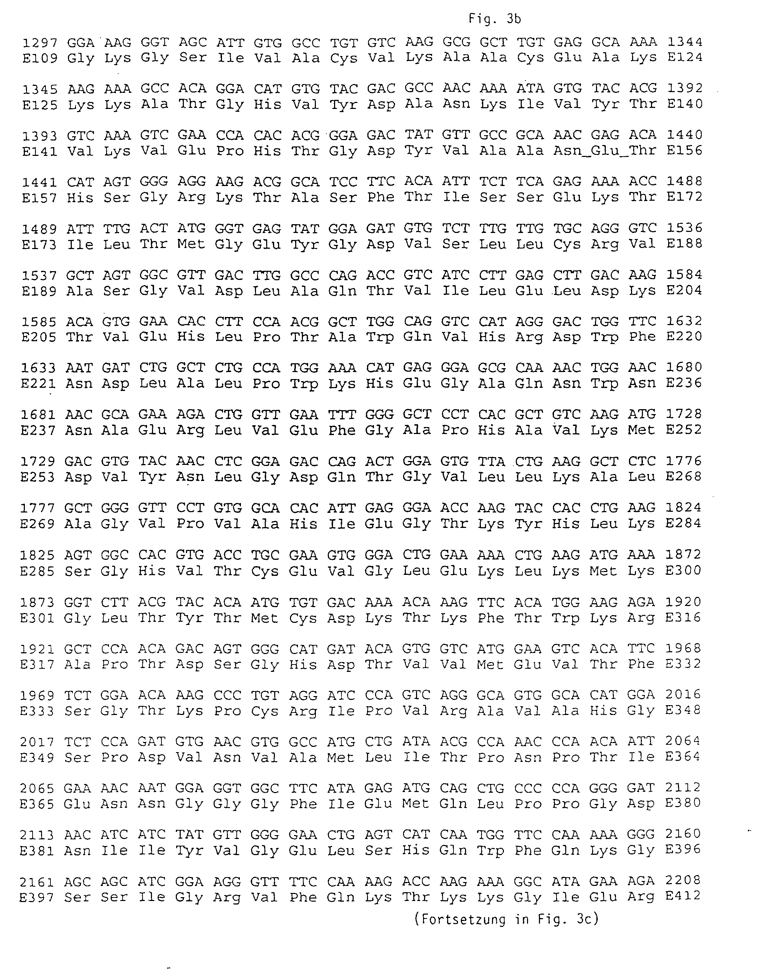

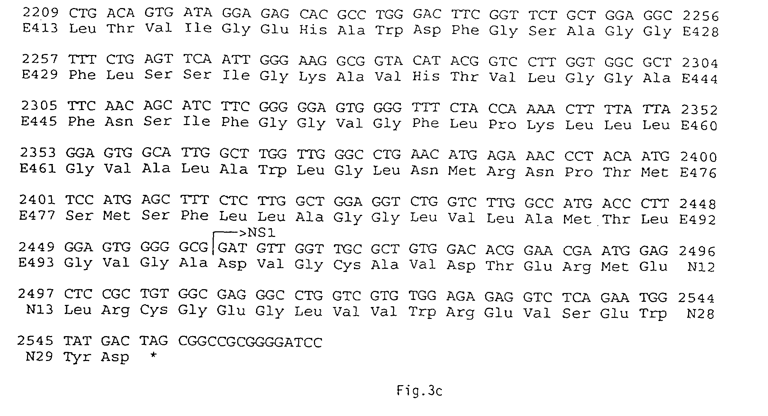

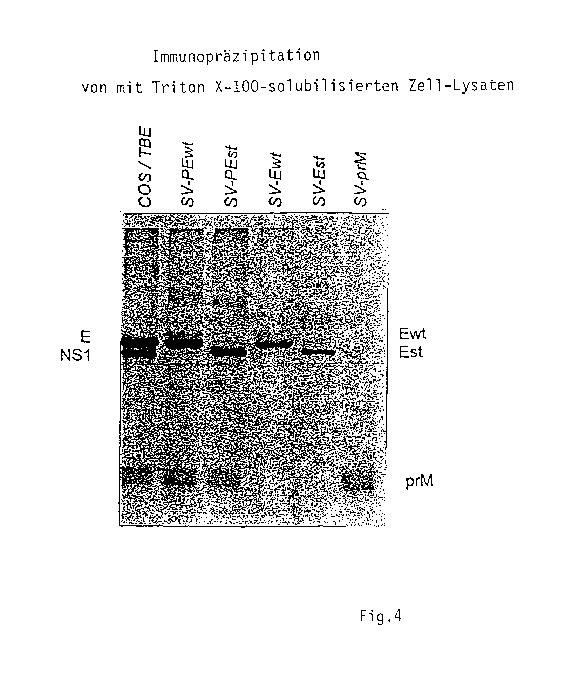

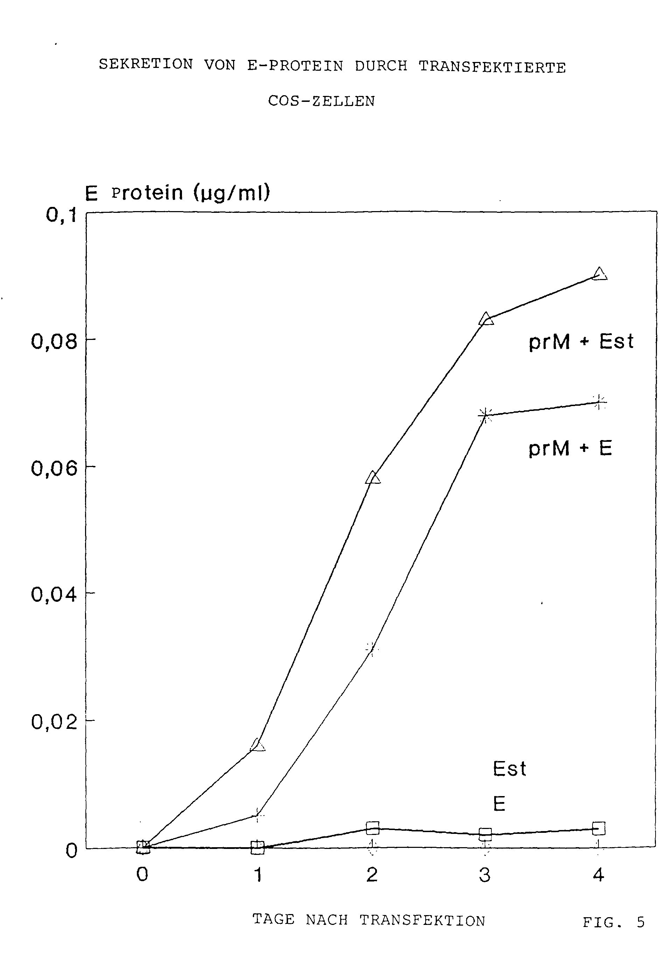

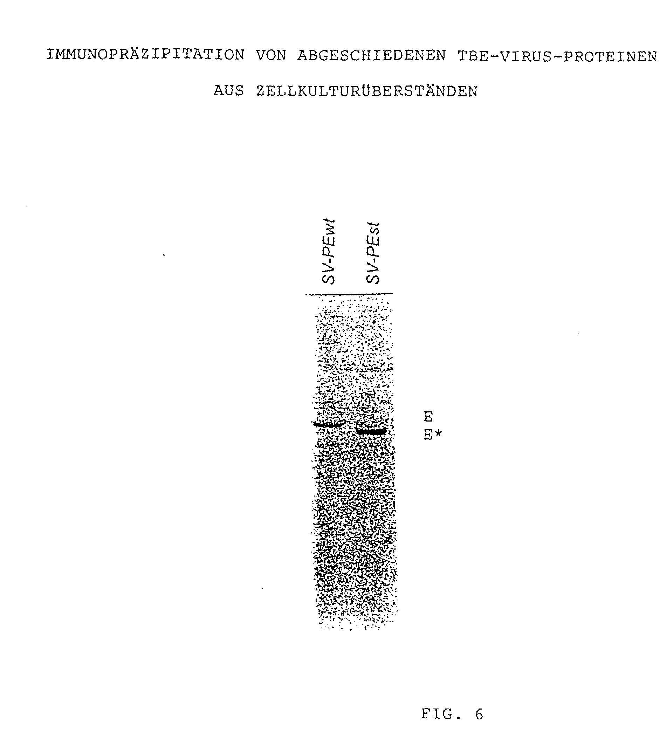

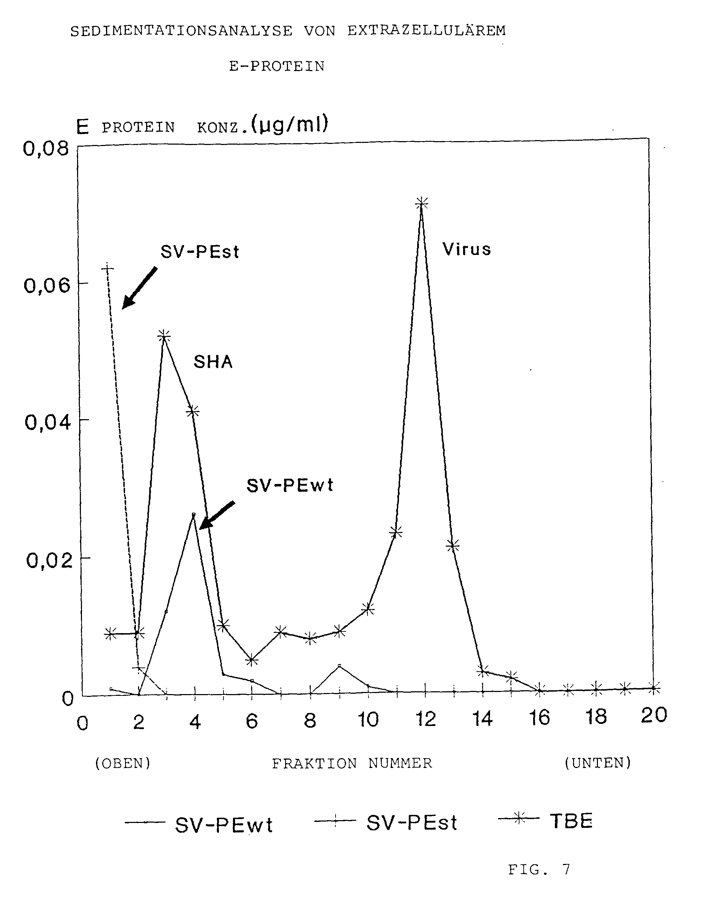

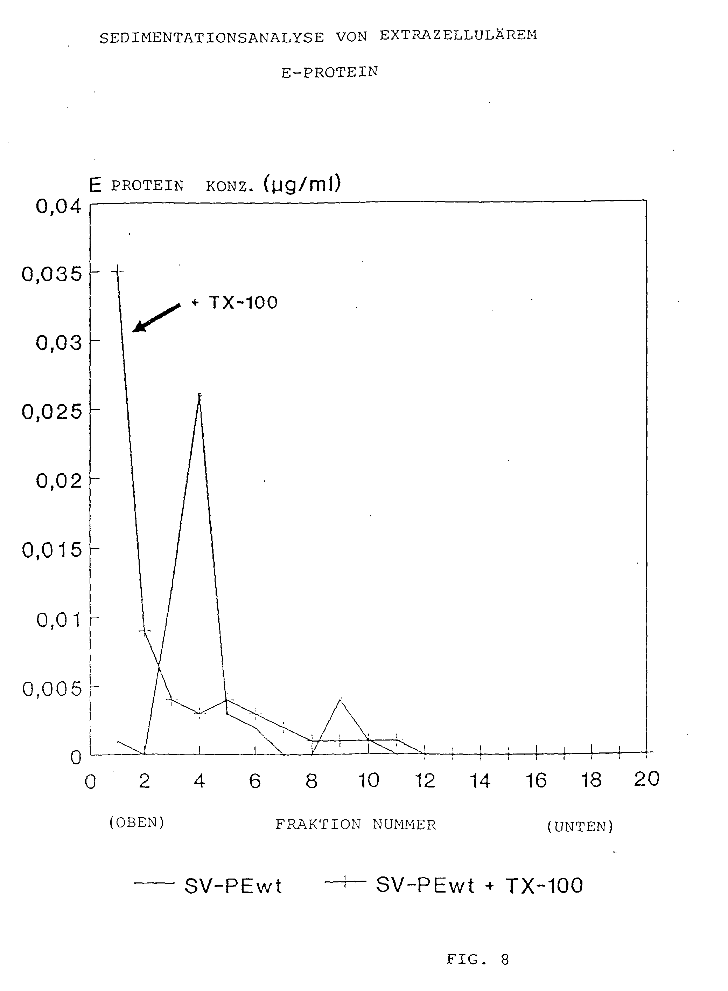

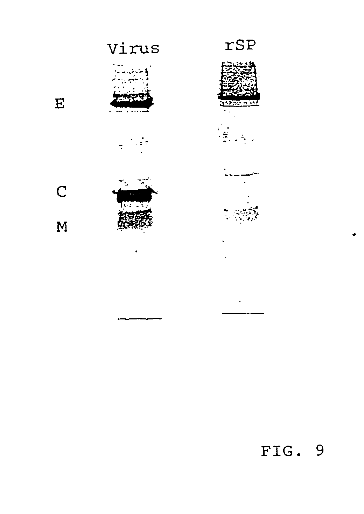

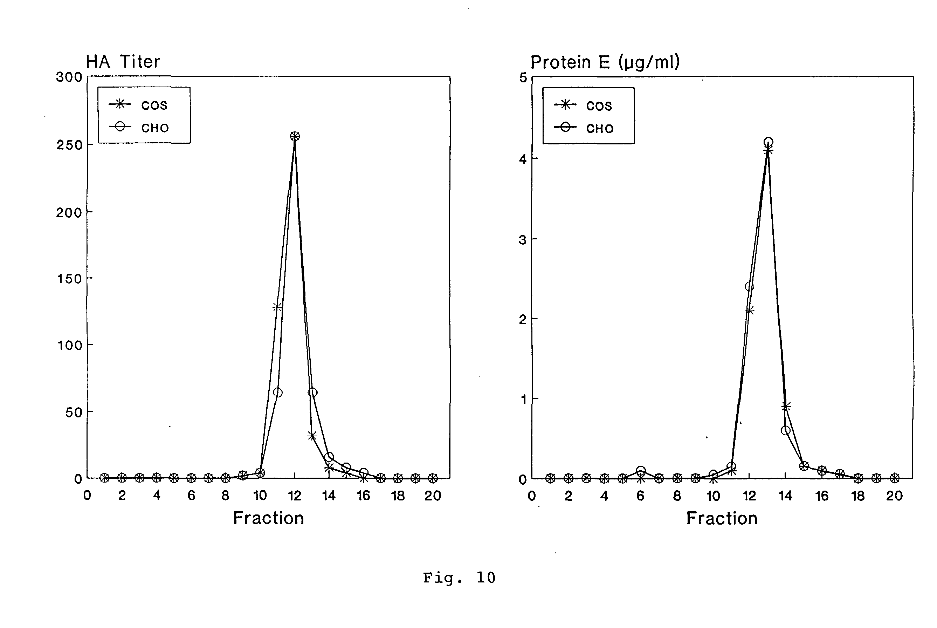

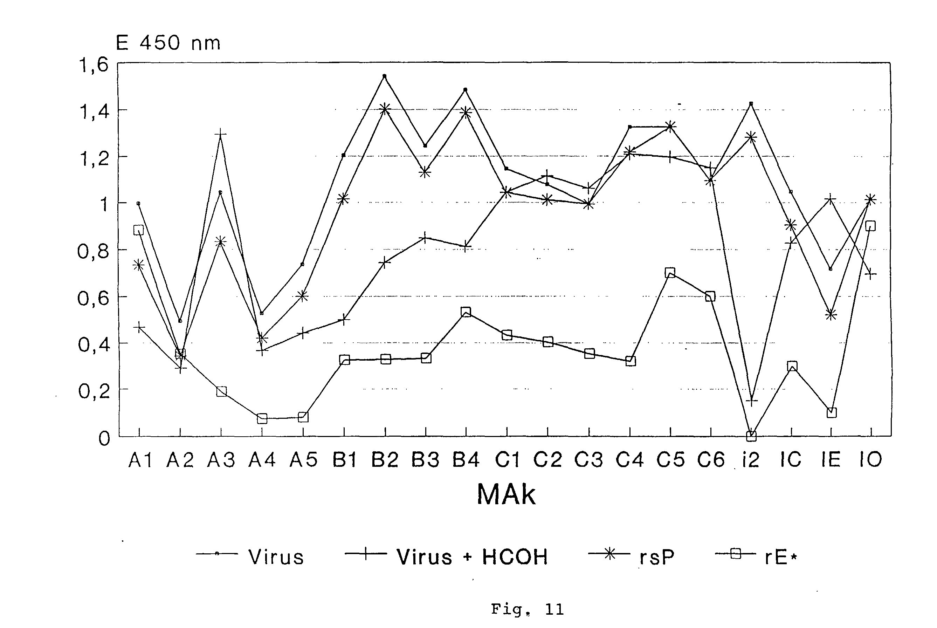

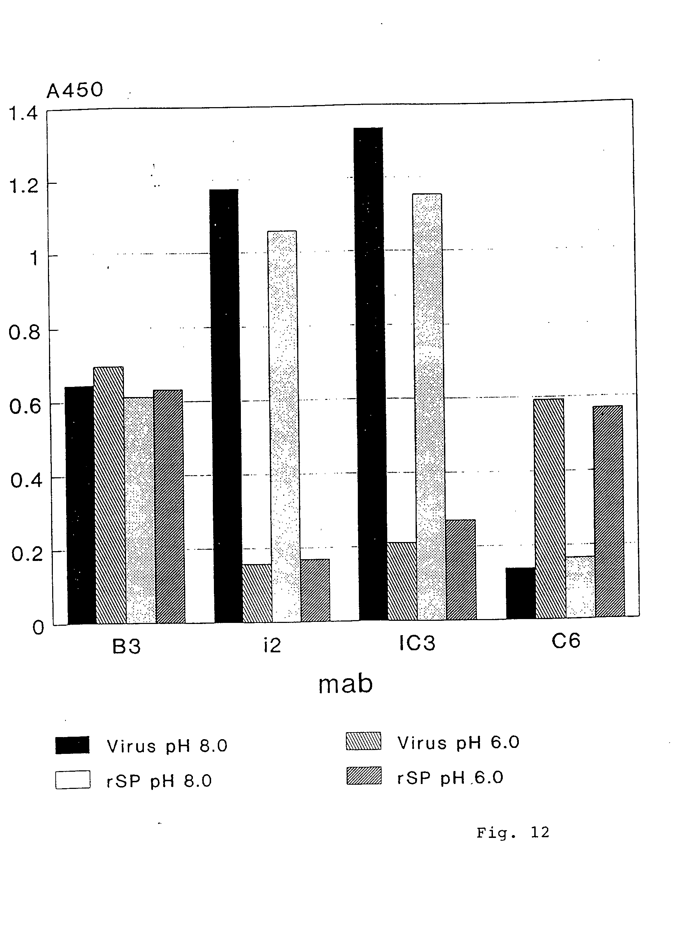

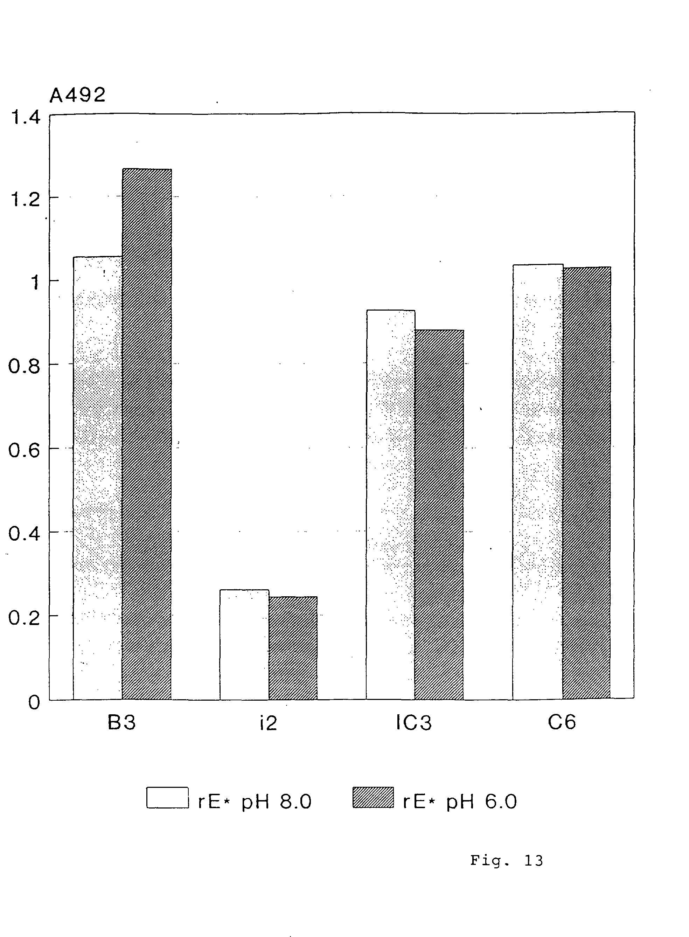

Die Erfindung wird nachstehend anhand von Ausführungsbeispielen unter Bezugnahme auf die Zeichnung noch weiter erläutert. Es zeigen: Fig. 1. eine schematische Darstellung der in den Expressionsplasmiden verwendeten Inserts, Fig. 2 eine schematische Darstellung des Plasmids pSVβ, das zur Expression der in Fig. 1 gezeigten Konstrukte verwendet wurde; Fig. 3a, b, und c eine vollständige Nukleotid- und Aminosäuresequenz des in Fig. 1 dargestellten Konstrukts SV-PEwt; Fig. 4 die Immunpräzipitation von mit Triton X-100 solubilisierten Zelllysaten nach Transfektion mit den in Fig. 1 dargestellten Konstrukten sowie nach Infektion mit TBE-Virus (COS/TBE); Fig. 5 in einem Diagramm den Nachweis von Protein E mittels 4-Schicht ELISA in den Zellkulturüberständen von COS-Zellen, die mit den in Fig. 1 dargestellten Konstrukten transfiziert worden waren; Fig. 6 die Immunpräzipitation des Zellkulturüberstandes von COS-Zellen nach Transfektion mit SV-PEwt bzw. SV-PEst Fig. 7 in einem Diagramm die Sedimentationsanalyse von COS-Zellkulturüberständen nach Transfektion mit SV-PEwt und SV-PESt sowie nach Infektion mit dem TBE-Virus. Die Sedimentationsrichtung ist von links nach rechts; Fig. 8 eine Sedimentationsanalyse von rSP ohne (SV-PEwt) bzw. nach Behandlung mit 0,5% Triton X-lOO. Die Sedimentationsrichtung ist von links nach rechts; Fig. 9 in einem Diagramm die SDS-PAGE von gereinigtem TBE-Virus und gereinigten rSPs; Coomassie-Blau Färbung; Fig. 10 den Vergleich von rSPs aus COS-Zellen mit jenen aus einer stabil transfizierten CHO-Zellinie; Fig. 11 in einem Diagramm die Reaktivität von 19 Protein Espezifischen monoklonalen Antikörpern im 4-Schicht ELISA mit TBE-Virus, Formalin-inaktiviertem TBE-Virus, rSP und rE*; Fig. 12 die Reaktivität von TBE-Virus bzw. rSP im 4-Schicht ELISA mit den mAks B3, i2, IC3 und C6 ohne (pH 8,0) bzw. nach Behandlung bei pH 6,0; und Fig. 13 die Reaktivität von pE* im 4-Schicht ELISA mit den mAKs B3, i2, IC3 und C6 ohne (pH 8,0) bzw. nach Behandlung bei pH 6,0.

Die Erfindung wird in den nachfolgenden Beispielen näher beschrieben.

### 1. Herstellung der Partikel

Zu diesem Zweck wurden 4 Expressionsplasmide konstruiert, die das E-Protein bzw. eine Membrananker-freie Form des E-Proteins allein bzw. gemeinsam mit prM enthalten (Fig.1).

Ein Beispiel für die Herstellung dieser Plasmide ist in Allison et al., Virus Genes 8 (3) (1994), Seiten 187-198 beschrieben.

Das dort beschriebene Ausgangsplasmid pSVβ ist in Fig.2 dargestellt.

Als Vektor wurde das pSV46 zur Konstruktion von Expressionsklonen verwendet. Dieser Vektor ist ein Derivat des auf SV40 basierenden eukaryontischen Expressionsvektors pSVβ (Clontech), von dem das das β-Galactosidasegen enthaltende Insert (MacGregor G.R. und Caskey C.T., Nucleic Acids Res 17, 2365, 1989) durch Verdau mit NotI und Religation entfernt worden war. Ein Teil des Polylinkers stromabwärts von der Translations-Stoppstelle wurde auch durch Verdau mit XbaI und HindIII, Klenow-Einfüllung und Religation entfernt.

In diesen Vektor wurden PCR-Produkte eingebaut, welche folgendermaßen erhalten wurden:

Synthetische Oligonucleotidprimer wurden zur Amplifizierung von Teilen der cDNA entsprechend entweder dem TBE-Virus prM+E-Bereich oder E allein verwendet. Die verwendeten Nukleotidkoordinaten sind ausgehend von Mandl et al. (Mandl C.W., Heinz F.X. und Kunz C., Virology 166, 197-205, 1988) dahingehend überarbeitet, daß sie die ersten 20 Nukleotide des TBE-Genoms (Mandl C.W., Heinz F.X., Stockl E. und Kunz C., Virology 173) enthalten. Die 5'-Primer für die prM+E- und nur-E-Konstrukte waren 27-mere mit den Sequenzen AAGCGGCCGCCATGGTTGGCTTGCAAA bzw. AAGCGGCCGCCATGGTTACCGTTGTGT. Die ersten 11 Nucleotide von jeder bestanden aus einer künstlichen Sequenz, enthaltend eine Erkennungsstelle für das Restriktionsenzym NotI (GCGGCCGC), gefolgt von einer 16-Nucleotidsequenz entsprechend entweder den Nucleotiden 388-403 (SV-PE-Reihe) oder 883-898 (SV-E-Reihe). In jedem Fall wurde ein natürlich vorkommendes ATG-Codon stromaufwärts des entsprechenden Gens als Startcodon verwendet, und der Primer war so gestaltet, daß er ATG in einem geeigneten Kontext (GCCGCCATGG) für effiziente Translationsinitiation in COS-Zellen aufweist (Kozak M., Cell 44, 283-292, 1986; Kozak M., J Mol Biol 196, 947-950, 1987). Das gleiche 28 Nukleotid-lange Oligonukleotid (ATGCGGCCGCTAGTCATACCATTCTGAG) wurde als 3'-Primer für beide Konstruktionen verwendet. Dieser Primer war an seinem 3'-Ende komplementär zu den Nucleotiden 2535-2550 und enthielt an seinem 5'-Ende eine NotI-Stelle und das Komplement (CTA) eines TAG-Stoppcodons im selben Leserahmen.

Die PCR-Amplifikation wurde unter im wesentlichen Standardbedingungen gemäß dem Cetus-Protokoll (Saiki R.K., Gelfand D. H., Stoffel 5., Scharf S.J., Higuchi R., Horn G.T., Mullis K.B. und Erlich H.A., Science 239, 487-491, 1988) durchgeführt. Die Reaktionsmischungen enthielten 50 mM KCl; 10 mM Tris-HCl, pH 8,3, 1,5 mM MgCl₂, jeweils 200 µM dNTP, 10 ng jedes Primers, 10 µl einer 1000fachen Verdünnung der cDNA-Matrize und 3 E AmpliTaq-Polymerase (Perkin Elmer Cetus) in einem Gesamtvolumen von 100 *µ*l. Die Proben wurden mit 100 µl Paraffinöl überdeckt und in einem Perkin Elmer Temperaturwechsler amplifiziert.

Die Proben wurden 6,5 min auf 94°C gehalten, dann auf eine Annealing-Temperatur von 40°C gekühlt, bevor Taq-Polymerase zugegeben wurde. Insgesamt wurden 35 Amplifikationszyklen durchgeführt. Jeder Zyklus bestand aus 1,5 min bei 94°C, 3 min bei 40°C und 7 min bei 68°C. Die Proben wurden mittels Phenol- und Chloroformextraktion gereinigt, Ethanol ausgefällt und in sterilem doppelt destilliertem Wasser wiedergelöst. Die Qualität und Quantität der PCR-Produkte wurde mittels Agarosegelelektrophorese ermittelt, und die Proben wurden bei -20°C aufbewahrt, bis sie für die anschließenden Klonierschritte gebraucht wurden.

Die Polymerase-Kettenreaktion wurde verwendet, um eine Minibank von cDNA-Plasmid-Klonen mit Inserts zu konstruieren, die entweder dem prM + E-Bereich (SV-PE-Reihe), Nucleotide 388-2550, oder dem E-Bereich (SV-E-Reihe), Nucleotide 883-2550, des TBE-Virus-Genoms entsprachen (Fig. 1).

Da die TBE-Virus-Strukturproteine normalerweise durch Co-Translationsprozessierung eines großen Polyprotein-Vorläufers erzeugt werden, wurden die Translations-Start- und -Stop-Stellen in die Enden der PCR-Produkte eingeführt, indem sie in die Oligonucleotid-Primer eingebaut wurden. In jedes Konstrukt wurde ein natürliches ATG-Codon, das in einem geeigneten Kontext placiert war, um als Initiator-Codon zu dienen, stromaufwärts der internen Signalsequenz eingeführt, um eine natürliche Prozessierung durch die Wirts-Signalase und das richtige Hinführen in den Sekretionsweg zu ermöglichen. Auf ähnliche Weise wurde das TG-Stop-Codon in jedem Konstrukt stromabwärts der Signalase-Spaltungsstelle, die den Carboxy-Terminus von Protein E bildet, plaziert. Die Erkennungsstellen für das Restriktionsenzym NotI wurden ebenfalls in die Enden eingebaut, um ein nachfolgendes Klonieren der PCR-Produkte zu erleichtern.

Die PCR-DNAs wurden mit NotI geschnitten und in die NotI-Klonierungsstelle des Plasmidvektors pSV46 ligiert, was ermöglichte, daß korrekt inserierte Gene unter der Steuerung des frühen SV40-Promotors exprimiert wurden (MacGregor G.R. und Caskey C.T., Nucleic Acids Res 17, 2365, 1989). Der Vektor stellte auch ein Polyadenylierungssignal für eine effiziente Expression und einen SV40-Replikationsursprung zur Verfügung, um eine Replikation der rekombinanten Plasmide in transfektierten COS-Zellen, die konstitutiv das große SV40-T-Antigen exprimieren (Gluzman Y., Cell 23, 175-182, 1981) zu ermöglichen.

Die Ligationsmischungen wurden zur Durchführung einer Transformation in E. coli HB 101 verwendet, und einzelne ampicillinresistente Kolonien, die einzelne Klone repräsentierten, wurden isoliert. Die Orientierung des Inserts in jedem Plasmid wurde durch Verdau mit Restriktionsenzymen und Agarose-Gelelektrophorese bestimmt. Klone mit richtig orientierten Inserts wurden zur weiteren Analyse behalten.

Plasmide mit richtig orientierten Inserts wurden durch Restriktionsanalyse identifiert und mittels CsCl-Gradientenzentrifugation gereinigt.

Für jedes Plasmidkonstrukt wurde gereinigte doppelsträngige Plasmid-DNA aus einer einzigen Präparation für die DNA-Sequenz-Bestimmung verwendet. Die Sequenzierungsreaktionen wurden in einem Perkin-Elmer-Temperaturwechsler unter Verwendung von TBE-spezifischen Primern, AmpliTaq-Polymerase (Perkin Elmer Cetus) und mit fluoreszierendem Farbstoff markierten Didesoxyterminatoren gemäß den Instruktionen des Herstellers (Applied Biosystems) durchgeführt. Die Produkte der Sequenzierungsreaktionen wurden auf einem automatischen DNA-Sequenzer 373A von Applied Biosystems analysiert.

### Analyse von PCR-erzeugten Mutationen

Um eine detaillierte Analyse der klonierten Inserts durchzuführen und die Effizienz der Mutagenese durch Taq-Polymerase zu beurteilen, wurden 13 einzelne E. coli-Klone, die Plasmide der SV-PE-Serie enthielten und 9, die Plasmide der SV-E-Serie enthielten, ausgewählt. Die Plasmid-DNA aus jedem Klon wurde gereinigt und durch vollständiges Sequenzieren der klonierten Inserts in beiden Strängen analysiert. Die cDNA-Sequenzen wurden dann mit der entsprechenden RNA-Sequenz des elterlichen Wildtyp-TBE-Virusstamms Neudörfl (Mandl C.W., Heinz F.X. und Kunz C., Virology 166, 197-205, 1988) verglichen.

Wie erwartet, enthielt jeder der Plasmid-Klone mehrere Nucleotidunterschiede in bezug auf die Wildtyp-TBE-Virussequenz. Mit einigen Ausnahmen (siehe unten) waren die meisten dieser Veränderungen Einzelmutationen, die offensichtlich während der PCR-Amplifikation eingeführt worden waren, und wurden nur in einem einzigen Klon gefunden.

**Tabelle 1.**

| Zusammenfassung PCR-erzeugter Mutationen | | | |
|---|---|---|---|
| | | Aminosäureänderungen^{b} | |
| Klon | Nucleotidänderungen^{a} | prM | E |
| SV-PE | | | |
| 01 | 945 G→A | | |
| | 1122 G→C | | Gln 50→His |
| | 1395 C→T | | |
| | 2027 T→C | | Val 352→Ala |
| | 2480 A→G^{c} | | |
| 02 | 551 T→A | Val 24→Glu | |
| | 1080 G→A | | |
| | 1153 T→C | | |
| | 1168 T→C | | Ser 66→Pro |
| | 1690 A→G | | Arg 240→Gly |
| | 2458 G→del | | Ala 495→Leserahmen-verschiebung |
| 03 | 952 T→C | Cys158→Arg | |
| | 976 C→T | | Arg 2→Cys |
| | 1163 A→G | | Lys 64→Arg |
| | 1679 A→G | | Asn 236→Ser |
| | 1889 T→A | | Met 306→Lys |
| 04^{d} | 1434 C→T | | |
| | 2275 A→T | | Lys 435→Stop |
| | 2364 G→A | | |
| 05^{e} | 701 T→C | Leu 74→Pro | |
| | 1488 C→A | | |
| | 1492 T→C | | |
| | 1893 T→A | | Cys 307―Stop |
| 06 | 782 T→C | Leu101→Pro | |
| | 865 A→G | Lys129→Glu | |
| | 1002 C→T | | |
| | 1972 G→A | | Gly 334→Arg |
| 07^{e} | 1327 G→del | | Ala 119→Leserahmen-verschieb. |
| | 2248 G→A | | Ala 426→Thr |
| 08 | 701 T→A | Leu 74→Gln | |
| | 2092 A→G | | Met 374→Val |
| | 2124 T→C | | |
| 09 | 452 T→C^{c} | | |
| | 960 A→T | | |
| | 1746 A→G | | |
| | 2086 A→G | | Ile 372→Val |
| | 2142 T→C | | |
| | 2290 G→A | | Val 440-Ilc |
| | 2361 A→G | | |
| SV-PE | | | |
| 10^{c} | 1625 A→G | | Asp 218→Gly |
| | 2475 T→C | | |
| 11^{c} | - | | |
| 12^{c} | 1203 G→A | | Met 77→Ile |
| 13^{c} | 1366 T→C | | Tyr 132→His |
| | 1381 A→G | | Ile 137→Val |
| | :723 G→A | | Met 252→Ile |
| SV-E | 2134 C→T | | |
| 01^{d} | 1239 C→T | | |
| 02 | 1093 A→T | | Met 41→Leu |
| | 2301 C→T | | |
| 03 | 1321 G→A | | Val 117-Ilc |
| | 1688 A→G | | Glu 239→Gly |
| | 1717 G→A | | Ala 249→Thr |
| | 2053 A→G | | Asn 361→Asp |
| | 2092 A→G | | Met 374→Val |
| 04 | 2073 T→C | | |
| | 2438 C→T | | Ala 489→Val |
| 05 | 938 T→C | (Leu153→Pro)^{f} | |
| | 973 T→C | | Ser 1→Pro |
| | 2401 T→G | | Ser 477→Ala |
| 06 | 891 C→T | | |
| | 1151 G→A | | Cys 60→Tyr |
| | 1364 T→C | | Val 131→Ala |
| | 1567 A→G | | Ile 199→Val |
| | 2045 T→C | | Ile 358→Thr |
| 07 | 1257 T→C | | |
| | 1694 T→C | | Leu 241→Pro |
| | 1794 A→G | | |
| | 2159 G→T | | Gly 396→Val |
| 08 | 1806 A→G | | |
| | 2205 A→G | | |
| 09 | 2491 A→dcl^{c} | | |

| | | | |
|---|---|---|---|
| ^{a}Nucleotidkoordinaten betreffen gesamtes TBE-Genom. | | | |
| ^{b}Aminosäurekoordinaten betreffen prM oder E. | | | |
| ^{c}Aminosäureänderungen in NS1 oder C-Gen. | | | |
| ^{d}SV-PE04 als "SV-PEst" umbenannt und SV-E01 als "SV-Ewt." umbenannt. | | | |
| ^{e}Andere nicht durch PCR-erzeugte Mutationen nicht angeführt (vgl. Text) | | | |
| ^{f}Nur der C-terminale Teil von prM war in diesem Konstrukt vorhanden | | | |

Wie in Tabelle 1 dargestellt, enthielten die 22 sequenzierten Inserts (43.549 bp insgesamt) 71 einzelne Nucleotidänderungen, die auf Taq-Polymerase-Fehler zurückzuführen waren. Von diesen Änderungen führten 42 (59%) zu Substitutionen in der vorausgesagten Aminosäuresequenz und 3 zu Deletionen, die zu Rahmenverschiebungen führten (Tabelle 2).

**Tabelle 2.**

| PCR-Mutationshäufigkeit | | |
|---|---|---|
| Basenpaaränderungen | Anzahl des Auftretens^{a} | Häufigkeit (pro bp) |
| G/C→A/T | 20 (28%) | 4,59 × 10⁻⁴ |
| G/C→T/A | 2 (2,8%) | 4,59 × 10⁻⁵ |
| G/C→C/G | 1 (1,4%) | 2,30 × 10⁻⁵ |
| A/T→G/C | 37 (52%) | 8,50 × 10⁻⁴ |
| A/T→C/G | 1 (1,4%) | 2,30 × 10⁻⁵ |
| A/T→T/A | 7 (9,9%) | 1,61 × 10⁻⁴ |
| G/C Deletion | 2 (2,8%) | 4,59 × 10⁻⁵ |
| A/T Deletion | 1 (1,4%) | 2,30 × 10⁻⁵ |
| Übergänge | 57 (80%) | 1,31 × 10⁻³ |
| Transversionen | 11 (15%) | 2,53 × 10⁻⁴ |
| Alle Mutationen | 71 (100%) | 1,63 × 10⁻³ |
| Aminosäuresubst. | 42 (59%) | 9,64 × 10⁻⁴ |
| Stille Mutationen | 24 (34%) | 5,51 × 10⁻⁴ |
| Leserahmenverschiebungen | 3 (4,2%) | 6,89 × 10⁻⁵ |
| Terminationscodons | 2 (2,8%) | 4,59 × 10⁻⁵ |

| | | |
|---|---|---|
| ^{a}43549 Basenpaare sequenziert | | |

Die Verteilung der Mutationen war stark zugunsten von A/T→G/C und G/C→A/T-Übergangsmutationen beeinflußt, wie bereits zuvor von anderen beobachtet worden war (Dunning A.M., Talmud P. und Humphries S.E., Nucleic Acids Res 16, 10393, 1988; Chen J., Sahota A., Stambrook P.J. und Tischfield J.A., Mutat Res 249, 169-176, 1991; Cadwell R.C. und Joyce G.F., PCR Methods Applic 2, 28-33, 1992). Die Gesamtmutationshäufigkeit war 1,63 x 10⁻³ pro Basenpaar bei einer 35-Zyklus-Amplifikation (4,7 x 10⁻⁵ pro Basenpaar pro Zyklus), was mit veröffentlichten Werten für die Fehlerhäufigkeit von Taq-Polymerase (15,32) übereinstimmt. Die durchschnittliche Aminosäuresubstitutionsrate war 0,46 pro Gen für prM (164 Aminosäuren lang) und 1,59 für E (496 Aminosäuren lang).

Eine stille Mutation am Nucleotid 930 war in allen Klonen vorhanden, und man nimmt an, daß sie als natürliche Mutation während der Virusvermehrung entstand. Außerdem wurde festgestellt, daß fünf Klone der SV-PE-Serie, SV-PE05, SV-PE07, SV-PE11, SV-PE12 und SV-PE13, sieben identische Mutationen gemeinsam hatten, an den Nukleotiden 849, 1285, 1346, 1404, 1909, 2247 und 2255. Mit Ausnahme von SV-PE10, der drei der Mutationen (1909, 2247 und 2255) enthielt, war keine dieser in den anderen SV-PE-Klonen oder irgendeinem der SV-E-Klone zu finden. Eine dieser Änderungen, eine Nucleotid-Deletion an der Position 1346, bewirkte eine Rahmenverschiebung am Codon 125 im E-Gen, und es wäre daher zu erwarten, daß sie ein nicht-funktionelles E-Protein ergeben. Da ein diese selben sieben Mutationen enthaltender Klon unabhängig von separaten cDNA- und PCR-Präparationen erhalten wurde (Daten nicht gezeigt), scheint es, daß diese Varianten bereits in der Virus-RNA-Population vor der Amplifikation vorhanden waren. Diese besonderen Mutationen wurden daher in der Analyse der PCR-erzeugten Mutationen nicht inkludiert.

### Konstrukte, die für Wildtyp- und deletierte E-Proteine codieren

Zusätzlich zu Klonen, die für einzelne Aminosäureänderungen codieren, waren wir auch an der Herstellung von prM + E und nur-E-Konstrukten, die für Wildtyp- und deletierte Proteine codieren, interessiert. Da jedoch alle der PCR-erzeugten Klone der SV-PE-Serie Mutationen enthielten, die zu Änderungen in der Aminosäuresequenz führen würden, wurde eine direkte Subklonierung verwendet, um ein Wildtyp-Konstrukt herzustellen, das für unmodifizierte prM und E-Proteine sowie für eine deletierte Form des nur-E-Konstrukts codiert.

Die DNA-Sequenz des Plasmids SV-PE04 zeigte außer zwei stillen Mutationen eine Substitution von A durch T am Nucleotid 2275, wodurch das AAG-Codon für Lysin 435 von Protein E zu einem TAG-Stop-Codon geändert wurde (Tabelle 1). Es wird daher vorhergesagt, daß SV-PE04 für ein Wildtyp-prM-Protein und ein deletiertes E-Protein codiert, dem die Carboxy-terminalen 61 Aminosäurereste fehlen, welche den mutmaßlichen Membran-durchspannenden Bereich (Mandl C.W., Guirakhoo F., Holzmann H., Heinz F.X. und Kunz C., J Virol 63, 564-571, 1989) beinhalten. Es zeigte sich, daß das Plasmid SV-E01, in welchem der Großteil des prM-Gens fehlt, nur eine stille Mutation im E-Gen enthielt, womit es für ein Wildtyp-E-Protein codiert.

Zur Schaffung eines prM + E-Konstrukts der gesamten Länge mit einer Wildtyp-Aminosäuresequenz und eines nur-E-Konstrukts ohne prM aber mit dem Stop-Codon am Nucleotid 2275, wurden Restriktionsfragmente aus SV-PE04 und SV-E01 (nachfolgend SV-PEst bzw. SV-Ewt genannt) ausgetauscht. Zu diesem Zweck wurden zwei Spaltstellen für das Restriktionsenzym Cfr10I verwendet, eine zwischen den Nucleotiden 960 und 961, nahe der Grenze der prM- und E-Gene, und die andere im Ampicillin-Resistenzgen des Vektors. Eines der resultierenden Plasmidkonstrukte, SV-PEwt, enthielt das Wildtyp-prM-Gen aus SV-PEst und das Wildtyp-E-Gen aus SV-Ewt, während dem anderen, SV-Est, das prM-Gen fehlte, dieses aber das Stop-Codon-enthaltende E-Gen aus SV-PEst enthielt. Diese Änderungen wurden durch Sequenzieren der Inserts beider Plasmide bestätigt. Diese beiden Konstrukte vervollständigten ein Set aus vier Expressionsplasmiden zum Vergleichen der Struktur und der Verarbeitung von aus einem Wildtyp-Konstrukt (SV-PEwt) exprimierten E-Proteinen mit jenen, welchen prM (SV-Ewt), der E-Ankerbereich (SV-PEst) oder beides (SV-Est) fehlte.

Die vollständige Sequenz des PEwt Inserts ist in Fig.3a, b und c dargestellt.

COS-Zellen wurden mit den geeigneten Plasmiden transfiziert und auf die Expression rekombinanter Proteine wie folgt analysiert:

### DNA-Transfektion

COS-1-Zellen (Gluzman Y., Cell 23, 175-182, 1981) wurden in "COS- Medium", bestehend aus Dulbecco's MEM (Gibco-BRL), ergänzt mit 10 % fötalem Rinderserum, Penicillin (100 E/ml) und Streptomycin (100 *µ*g/ml) bei 37°C und 5 % CO₂ gehalten.

Plasmide, enthaltend klonierte TBE-Gene, wurden durch liposomvermittelte Transfektion unter Verwendung einer Modifikation des Verfahrens von Felgner et al. (Felgner P.L., Gadek T.R., Holm M. Roman R., Chan H.W., Wenz M., Northrop J.P., Ringold G.M. und Danielsen M., Proc Natl Acad Sci USA 84, 7413-7417, 1987) mittels eines BioRad Gene Pulser-Apparates in COS-1-Zellen eingebracht. Lipofectin-Reagens (Gibco-BRL) wurde in Opti-MEM-I-reduziertem Serummedium (Gibco-BRL) auf 20 *µ*g/ml verdünnt und mit einem gleichen Volumen an OptiMEM-I, enthaltend 8 *µ*g/ml der entsprechenden Plasmid-DNA gemischt. Pro Transfektion wurden 5 *µ*g Lipofectin und 2 *µ*g Plasmid-DNA verwendet. Die Mischung wurde 15 min bei Raumtemperatur zur Bildung von DNA-Lipofectin-Komplexen stehengelassen, und 0,5 ml dieser Mischung wurden in jede Vertiefung einer Zellkulturplatte (Nunc) mit 24 Vertiefungen, enthaltend Zellen, die zur Entfernung von Spuren von Serum aus dem Wachstumsmedium zweimal mit 1 ml OptiMEM gewaschen worden waren, zugegeben. Die Zellen wurden mit der DNA-Lipofectin-Mischung 5 h bei 37°C, 5 % CO₂, inkubiert, gefolgt von der Zugabe von 1 ml komplettem COS-Medium, und die Inkubation'wurde über Nacht fortgesetzt. 24 h nach der Transfektion wurde das Medium entfernt und durch frisches COS-Medium ersetzt, und die Inkubation wurde fortgesetzt bis ca. 46 h nach der Transfektion, zu welchem Zeitpunkt Zellen entweder zur Immunfluoreszenzanalyse fixiert oder zur Analyse auf intrazelluläres Antigen mittels ELISA aufgelöst waren.

Das Verfahren zur Infektion von COS-Zellen mit TBE-Virus für Immunfluoreszenz-Kontrollen war im wesentlichen identisch mit dem Transfektionsverfahren, mit der Ausnahme, daß anstelle von Plasma-DNA und Lipofektin TBE-Virus in Form einer Gehirnsuspension von Mäusesäuglingen, die 100fach in Opti-MEM-I-Medium verdünnt war, verwendet wurde.

### Analyse der exprimierten Proteine

### a. Immunopräzipitation von Zell-Lysaten

Transfizierte Zellen wurden mit ^{3 5}S-Cystein markiert, mit 1% Triton X-100 solubilisiert und einer Immunpräzipitation mit einem polyklonalen Kaninchenserum, das für TBE-Virus E und prM-Proteine spezifisch war, unterzogen. Wie aus Fig. 4 ersichtlich, führte die Expression der Konstrukte SV-PEwt und SV-Ewt zur Synthese von Protein E authentischer Größe. Die aus SV-PEt und SV-Est synthetisierten Proteine waren, wie erwartet, infolge ihrer C-terminalen Verkürzung etwas kleiner als das Wildtyp-E-Protein.

### b. Analyse der Sekretion von rekombinantem Protein im Zellüberstand

Überstände von transfizierten Zellen wurden quantitativ auf das Vorhandensein von Protein E an den Tagen 0 bis 4 nach der Transfektion unter Verwendung eines vierschichtigen ELISA, wie in Heinz et al., J.Biol.Stand. (1986), 14: 133-141 beschrieben, analysiert. Die in Fig. 5 dargelegten Ergebnisse zeigen, daß nur jene Konstrukte, die das prM-Protein co-exprimieren, zur Sekretion von Protein E führten. Immunopräzipitation aus den Überständen (Fig. 6) bestätigte, daß die abgeschiedenen Proteine dieselbe Größe hatten wie die entsprechenden intrazellulären Proteine (vgl. Fig. 4).

Die Sekretion des rekombinanten Proteins stellt einen enormen Vorteil für Produktionszwecke dar, da damit die Notwendigkeit der Lyse der Zellen zur Gewinnung des erwünschten Proteins und des Entfernens von kontaminierendem Zellmaterial eliminiert wird. Unter geeigneten Bedingungen ermöglicht dies auch die kontinuierliche Ernte des rekombinanten Proteins, ohne die Zellen zu zerstören.

### c. Charakterisierung von abgeschiedenen rekombinanten Proteinen

Die in Fig. 5 gezeigten Überstände wurden einer Saccharose-Dichtegradienten-Zonalzentrifugation unter Verwendung von 5 bis 30% (Masse/Masse) Saccharose-Gradienten und eines Beckman SW-40-Rotors unterzogen. Die Zentrifugation erfolgte bei 38000 U/min und 4°C 100 minuten lang. Die Gradienten wurden fraktioniert, und Protein E wurde in jeder Fraktion mittels vierschichtigem ELISA (s. Heinz et al., J.Biol.Stand. (1986) 14: 133-141) quantitativ bestimmt. Der Überstand aus Virus-infizierten Zellen wurde als Kontrolle verwendet, welche zwei Protein-E-hältige Peaks (Fig. 7) ergab, wovon einer dem kompletten Virus und einer dem sogenannten nicht-infektiösen "langsam sedimentierenden Hämagglutinin" ("slowly-sedimenting hemagglutinin" (SHA)) (s. Russel et al. (1980), Chemical and antigenic structure of flaviviruses. In: Schlesinger, R.W. (ed.) The Togaviruses, 503-529, Academic Press) entsprach. Der Überstand aus den mit SV-PEwt transfizierten Zellen enthielt eine partikuläre Form von E mit einer Sedimentationsgeschwindigkeit ähnlich dem viralen SHA, während das C-terminal verkürzte Protein E von SV-PEst keine stabilen Teilchen bildete und oben auf dem Gradienten verblieb.

Die partikuläre Form aus SV-PEwt wurde "rekombinantes subvirales Teilchen" ("recombinant subviral particle", rSP) genannt, und die lösliche Form aus SV-PEst "rekombinantes E*"(rE*).

Eine Behandlung von rSP mit 0,5% Triton X-100 dissoziierte die Teilchen, wie durch die Sedimentationsanalyse von Fig. 8 gezeigt, was auf die Anwesenheit einer Lipidmembran hindeutet.

### Präparation von rSP und rE*

### a. Reinigung von rSP

Überstände von mit SV-PEwt transfizierten COS-Zellen wurden durch 3Ominütige Zentrifugation bei 10000 U/min bei 4°C in einer Sorvall-Hochgeschwindigkeitszentrifuge geklärt, und die Teilchen wurden dann durch Zentrifugieren bei 44000 U/min, 4°C, während 120 min geerntet, wobei ein Beckman Ti45 Rotor verwendet wurde. Die rSP-hältige Pellet-Fraktion wurde in TAN-Puffer, pH 8,0, resuspendiert und auf einen 5-20% Saccharose-Gradienten, der mit demselben Puffer erzeugt worden war, aufgetragen. Nach 90minütiger Zentrifugation bei 38000 U/min und 4°C unter Verwendung eines Beckman SW4O-Rotors wurden die Proben fraktioniert, und die Peak-Fraktionen wurden durch Testen auf HA-Aktivität (Clarke und Casals, 1958, Aver.J.Trop.Med.Hyg. 7: 561-573) identifiziert. Peak-Fraktionen aus dem Zonalgradienten wurden mittels Gleichgewichts-Zentrifugation (35.000 U/min, 4°C, über Nacht) weiter gereinigt, und die Fraktionen wurden wiederum mittels HA identifiziert. Gesamtprotein E wurde mittels 4-schichtigem ELISA quantitativ bestimmt, und die Reinheit wurde unter Verwendung von Coomassie-Blau-Färbung mittels SDS-PAGE bestimmt (Fig. 9).

### b. rE*-Präparation

Serum-freie Überstände aus SV-PEst-transfizierten COS-Zellen wurden wie oben beschrieben geklärt und durch Ultrafiltration etwa 15-fach konzentriert.

### Herstellung von Zellinien mit chromosomal integrierten prM und E-Genen

Zur Herstellung von stabil transfizierten Zellen, die rSPs produzieren, wurde das Dihydrofolatreduktase (DHFR)-Selektionssystem verwendet (Current Protocols in Molecular Biology, John Wiley & Sons, Inc.) sowie jenes Konstrukt, das die vollständigen Gene für das prM und das E-Protein enthält (Fig. 1) und zur Synthese und Sekretion von rSPs führt. Der Transfer des DHFR-Gens erfolgte mit Hilfe des Plasmids pSV2-dhfr (S. Subramani et al., Mol.Cell.Biol. 1, 854-864, 1981), der Transfer der prM- und E-Gene mit Hilfe des Plasmids CMV-PEwt, das durch Umklonierung des Inserts aus dem Plasmid SV-PEwt in das Plasmid pCMVβ (Clontech) hergestellt wurde.

Die DHFR-defiziente Hamsterzellinie CHO-DG44 (Som.Cell.Mol.Get. 12, 555-666, 1986) wurde HAM's F12-Medium + 10 % fötalem Kälberserum (FKS) gezüchtet und mit pCMV-PEwt sowie pSV2-dhfr im Verhältnis 20:1 durch Elektroporation unter Verwendung eines BioRad Gene Pulser-Apparates transfiziert. Die transfizierten Zellen wurden zunächst 2 Tage in HAM's F12-Medium + 10 % FKS weitergezüchtet, dann trypsinisiert und in hoher Verdünnung zur Gewinnung einzelner, DHFR und prM+E exprimierender Zellklone in Selektionsmedium (MEM-α ohne Ribo- und Deoxyribonucleotide + 10 % dialysiertes FKS) in Petrischalen ausgesät. Nach ca. 10 Tagen wurden einzelne im Mikroskop sichtbare Zellkolonien in kleine Zellkulturgefäße transferiert und weitervermehrt. Jene Zellklone, die FSME-spezifisches Antigen produzierten, wurden durch Analyse der Zellkulturüberstände mittels ELISA identifiziert.

Einer dieser Zellklone (CHO-37) wurde für die Gewinnung und Charakterisierung der von ihm produzierten rSPs verwendet. Dazu wurden die Zellen in obigem Selektionsmedium gezüchtet und die rSPs im Zellüberstand dem gleichen Reinigungsverfahren unterzogen, wie es für die aus COS-Zellen gewonnenen rSPs beschrieben ist. Die Fig. 10 stellt das Ergebnis einer Saccharose-Dichtegradientenzentrifugation dar, bei der die rSPs aus COS-Zellen mit jenen aus der stabil transfizierten Zellinie CHO-37 verglichen wurden. Folgende Zentrifugationsbedingungen wurden verwendet: 20-50 % Saccharose, Beckman Ti-40-Rotor, 35000 UpM über Nacht. Nach der Zentrifugation wurden die Gradienten fraktioniert und die einzelnen Fraktionen im Hämagglutinationstest und ELISA auf FSME Virus-spezifisches Antigen analysiert. Wie aus der Figur hervorgeht, wiesen die beiden Präparationen keine Unterschiede im Sedimentationsverhalten bzw. in der spezifischen Dichte sowie in der spezifischen Hämagglutinationsaktivität auf.

### 2. Charakterisierung in Bezug auf native Proteinstruktur

Die solcherart hergestellten Präparationen wurden den folgenden Analysen unterzogen:
a. Analyse der Antigenstruktur mit monoklonalen Antikörpern
b. Fähigkeit zu säureinduzierten Konformationsänderungen
c. Hämagglutinationsaktivität

### zu a. Antigenstruktur

Die Antigenstruktur des Formalin-inaktivierten Ganzvirus, von rSP und rE* wurde mit jener des nativen infektiösen Virus unter Verwendung von 19 Protein-E spezifischen monoklonalen Antikörpern verglichen (Mandl et al. 1989, J. Virol. 63: 564-571; eigene Versuche). Die Reaktivität jedes einzelnen monoklonalen Antikörpers mit jeder der obigen Präparationen wurde in einem 4-Schichten ELISA wie von Heinz et al., J.Biol.Stand. (1986), 14: 133-141 beschrieben, analysiert. Dabei wurden alle Antigenpräparationen in einer vorher bestimmten, konstanten Konzentration des Proteins E von 1 *µ*g/ml und jeder der monoklonalen Antikörper in einer Verdünnung eingesetzt, die mit dem nativen, infektiösen Virus einen Extinktionswert im Bereich von 0,5 bis 1,6 ergab.

Wie aus der Fig. 11 hervorgeht, ist das Reaktivitätsmuster mit den mAks bei rSP und dem infektiösen Virus praktisch identisch, so daß davon ausgegangen werden kann, daß das Protein E in den rSPs in derselben nativen Form wie auch am infektiösen Virus vorliegt.

Die Formalininaktivierung hingegen bewirkt signifikante Veränderungen in der Antigenstruktur, von der auch Epitope betroffen sind, die neutralisierende Antikörper binden. Dies betrifft sowohl den neutralisierenden mAk i2 (Mandl et al., 1989, J.Virol. 63: 564-571), dessen Epitop durch Formalinbehandlung zerstört wird, als auch Epitope im Bereich der Domäne A sowie in der Domäne B (Mandl et al., 1989, J.Virol. 63: 564-571), die zumindest eine verringerte Reaktivität aufweisen. Diese Analyse zeigt auch, daß E* in seiner Struktur keineswegs dem nativen Protein E an der Virusoberfläche entspricht.

### zu b.) Säureinduzierte Konformationsänderungen

Das TBE-Virus dringt mit Hilfe von Rezeptor-vermittelter Endocytose in Zellen ein und gelangt dadurch in Endosomen, deren saurer pH (<6,4) eine spezifische Konformationsänderung induziert, die für die Fusion der Virusmembran mit der Endosomenmembran und damit für die Infektiosität des Virus erforderlich ist. Diese strukturellen Veränderungen können durch die Änderung der Reaktivität einzelner mAks verfolgt werden (Heinz et al., 1994, Virology 198: 109-117) und betreffen unter anderem die Epitope i2 und IC3 (starke Reduktion der Reaktivität) bzw. C6 (verstärkte Reaktivität), nicht aber das Epitop B3.

Diese Veränderungen sind für die Funktion des Proteins E essentiell. Die Fähigkeit, auf sauren pH in der beschriebenen Weise zu reagieren, stellt daher ebenfalls ein Kriterium dafür dar, ob das Protein E in einer nativen, dem infektiösen Virus entsprechenden Form vorliegt. Präparationen von rSP, rE* und infektiösem Virus in Triethanolaminpuffer pH 8,0 wurden mit Hilfe eines Puffers enthaltend 0,05 m mES, 0,1 m NaCl und 0,1% Rinderalbumin gemischt, so daß ein pH-Wert von 6,0 entstand, 10 min. bei 37°C inkubiert und dann mit Hilfe von Triethanolamin wieder auf pH 8,0 eingestellt. Dann wurde die Reaktivität dieser Präparationen vor und nach Inkubation bei saurem pH mit den mAKs B3, i2, IC3 und C6 in einem 4-Schicht ELISA wie unter "Antigenstruktur" beschrieben analysiert. Die Ergebnisse sind in den Fig. 12 und 13 dargestellt und zeigen, daß saurer pH im Protein E der rSPs die gleichen strukturellen Veränderungen verursacht wie im infektiösen Virus (s. Fig.12), daß aber die gleiche Analyse bei rE* keinerlei Hinweise auf ähnliche Umlagerungen ergibt (s. Fig.13).

### zu c) Spezifische Hämagglutinationsaktivität

Eine charakteristische Eigenschaft des infektiösen FSME-Virus ist seine Fähigkeit, unter bestimmten Bedingungen Gänseerythrozyten agglutinieren zu können (Hämagglutinationsaktivität). Diese Eigenschaft wird dem Virus durch das Protein E vermittelt und ist somit ein weiterer Indikator für das Vorliegen einer nativen, funktionellen Struktur dieses Proteins. Hämagglutinationstests wurden wie von Clarke und Casals (1958), Amer.J.Trop. Med.Hyg. 7: 561-573 beschrieben unter Verwendung von Gänseerythrozyten bei einem pH von 6,4 durchgeführt.

Infektiöses Virus, Formalin-inaktiviertes Virus, rSP und rE* wurden auf einen im ELISA bestimmten Antigengehalt von 5 *µ*g/ml eingestellt und im Hämagglutinationstest analysiert. Das Ergebnis ist in der nachfolgenden Tabelle 3 dargestellt. Daraus geht hervor, daß rSP die gleiche spezifische Hämagglutinationsaktivität besitzt wie infektiöses Virus, und daß diese durch Protein E ver mittelte Aktivität bei der Formalininaktivierung fast vollständig verloren geht. Die Präparation rE* besitzt keinerlei meßbare HA- Aktivität.

**Tabelle 3**

| HA-Aktivität | |
|---|---|
| Präparation | HA-Titer |
| infektiöses Virus | 512 |
| Formal-inakt. Virus | 4 |
| rSP | 512 |
| rE* | < 2 |

### 3. Immunogenität in Mäusen

Die Immunogenität folgender Antigen-Präparationen wurde durch Immunisierung von Mäusen analysiert:
- Formalin-inaktiviertes gereinigtes Virus
- rSP
- rE*

Der Gehalt an Protein E wurde in einem Enzymimmunoassay ermittelt (Heinz et al., 1986, J.Biol-.Stand, 14: 133-141) und alle Präparationen wurden auf dieselbe Antigenkonzentration von 5 *µ*g/ml eingestellt. Entsprechend der derzeit verwendeten TBE-Vakzine (FSME-Immun®) wurde als Verdünnungspuffer PBS pH 7,4, enthaltend 0,1% Humanalbumin verwendet und 0.2% Al(OH)₃ als Adjuvans zugegeben.

Die Immunogenität von rSP und rE* wurde auch ohne Zugabe von Adjuvans getestet.

### Immmunisierungsprotokoll :

Mit jeder der Präparationen wurden Gruppen von je zehn 15 g schweren Swiss albino Mäusen (5 Weibchen und 5 Männchen) zweimal im Abstand von 14 Tagen subcutan immunisiert, wobei pro Maus und Immunisierung 0.2 ml des Antigens verabreicht wurden. Eine Woche nach der zweiten Immunisierung erfolgte die Blutabnahme und alle Mäuse wurden mit 500 LD₅₀ des mäusepathogenen TBE-Virusstammes Hypr intraperitoneal infiziert (Challenge Test). Die Mäuse wurden über einen Zeitraum von 14 Tagen auf das Auftreten einer zum Tode führenden Enzephalitis beobachtet.

Identische Aliquots des Serums jeder einzelnen Maus aus einer 10er Gruppe, die mit dem selben Immunogen imununisiert worden waren, wurden gepoolt und der TBE-Virus-spezifische Antikörpergehalt im Enzymimmunoassay (ELISA), Hämagglutinationshemmungstest (HHT) und Neutralisationstest analysiert.

Der ELISA wurde unter Verwendung von gereinigtem TBE-Virus als Antigen wie von Heinz et al. (1984), J.Gen.Virol. 65: 1921-1929 beschrieben durchgeführt. Der HHT erfolgte wie von Clarke und Casals (1958), Amer.J.Trop.Med.Hyg. 7: 561-573 beschrieben, wobei Gänseerythrozyten bei einem End-pH von 6,4 eingesetzt wurden. Für den Neutralisationstest wurden BHK-21 Zellen und eine Viruskonzentration von 500 infektiösen Einheiten verwendet.

Die Ergebnisse des Immunisierungsversuches sind in der nachstehenden Tabelle 2 zusammengefaßt. Daraus geht hervor, daß rSP sowohl ohne als auch mit Adjuvans genauso wie das Formalin-inaktivierte Ganzvirus alle Mäuse vor einer tödlichen Dosis des TBE-Virus schützte. Im Gegensatz dazu war nach Immunisierung mit rE* keine (ohne Adjuvans) oder nur eine minimale Schutzwirkung (mit Adjuvans) zu beobachten.

In Bezug auf die Antikörperinduktion waren die rSPs dem Formalin-inaktivierten Virus sogar überlegen, besonders deutlich bei Verwendung des Al(OH)₃ Adjuvans. Dies betrifft sowohl die im ELISA nachgewiesenen Virus-bindenden Antikörper als auch diefür eine schützende Immunantwort noch bedeutenderen - im HHT und NT gemessenen Antikörper, die also spezifische Funktionen des Virus (Hämagglutination bzw. Infektiosität) blockieren.

In Übereinstimmung mit der nicht vorhandenen bzw. geringen protektiven Wirksamkeit war auch die Antikörperinduktion durch rE* sehr niedrig bzw. nicht nachweisbar.

Es zeigt sich also, daß rSPs hervorragende Immunogene darstellen, die vor der ansonsten tödlich verlaufenden Enzephalitis schützen und in Bezug auf die Induktion funktioneller, virusneutralisierender Antikörper, dem Formalin-inaktivierten Ganzvirus sogar überlegen sind.

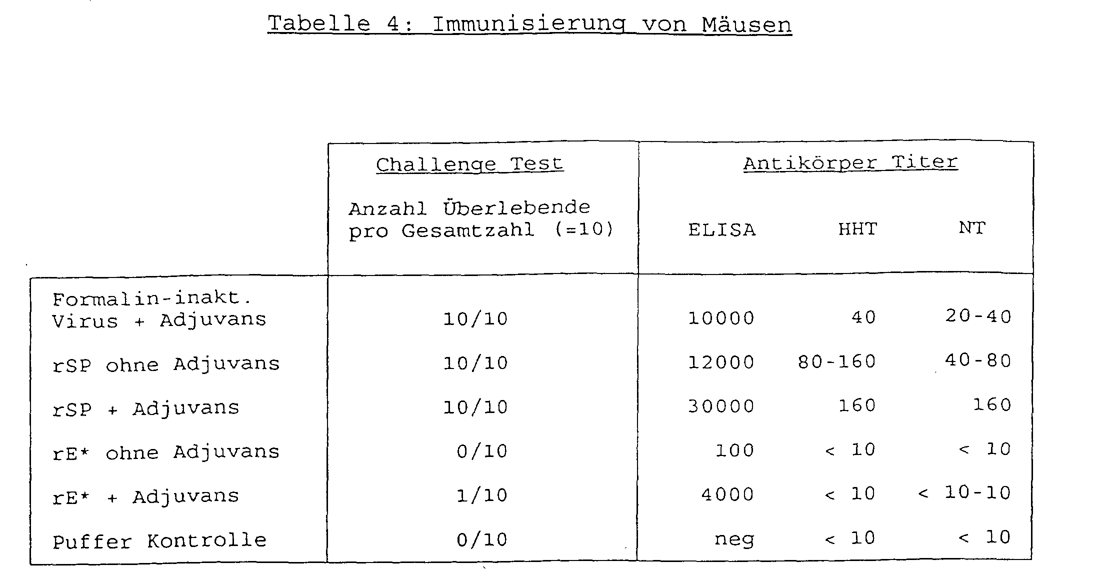

### 4. Immunisierung mit nackter DNA

Zur Immunisierung mit nackter DNA wurden Plasmide verwendet, welche die in Fig. 1 dargestellten Inserts unter der Kontrolle des CMV-"Immediate Early"-Promotors enthielten. Diese Plasmide (CMV-PE wt, CMV-PEst, CMV-Ewt, CMV-Est) wurden durch Umklonierung der Inserts aus den beschriebenen Plasmiden SV-PEwt, SV-PEst, SV-Ewt und SV-Est in das Plasmid pCMVβ (Clontech) gewonnen (Mac Gregor et al., Nucleic Acids Research, IRL Press, Vol. 17, Nr. 6, 1989, S. 2365: "Construction of plasmids that express E. coli β-galactosidase in mammalian cells") und durch CsCl-Dichtegradientenzentrifugation gereinigt (Maniatis et al., Molecular Cloning: A Laboratory Manual. Cold Spring Harbor Laboratory, Cold Spring Harbor, N.Y. 1982)

### CMV-PEwt enthält die Sequenzen für die Wild-Typ-Proteine prM und E.

CMV-PEst enthält die Sequenzen für das Wild-Typ-Protein prM und eine deletierte Sequenz des Proteins E, wobei die Codons für 61 carboxyterminale Aminosäuren, welche den Membrananker des Proteins E bilden, fehlen.

In CMV-Ewt ist der Großteil der prM-Sequenz nicht vorhanden, jedoch die vollständige Sequenz des Wild-Typ-Proteins E.

CMV-Est enthält keine prM-Sequenz und die deletierte Protein E-Sequenz aus CMV-PEst.

Jedes dieser vier Plasmide wurde in zwei Konzentrationen (60 *µ*g/ml und 300 *µ*g/ml in PBS) zur Immunisierung von Mäusen (Swiss albino) eingesetzt, wobei für jede Plasmidpräparation 10 Mäuse (5 Weibchen und 5 Männchen) verwendet wurden. Jeder Maus wurden 2 mal 100 *µ*l der jeweiligen DNA-Präparation im Abstand von 2 Wochen intradermal injiziert. Als Kontrollen wurden das Plasmid pCMVβ in einer Konzentration von 300 *µ*g/ml sowie PBS in gleicher Weise verwendet. Zum Vergleich mit der konventionellen Immunisierung wurden Gruppen von je 10 Mäusen mit dem Formalin-inaktivierten Virus in Konzentrationen von 1 *µ* g/ml und 5 *µ*g/ml ebenfalls 2 mal im Abstand von 2 Wochen immunisiert, wobei 0,2 ml pro Maus subkutan injiziert und 0,2 % Al(OH)₃ als Adjuvans verwendet wurden. Eine Woche nach der zweiten Immunisierung wurde den Mäusen zum Nachweis spezifischer Antikörper im ELISA Blut abgenommen, und gleichzeitig erfolgte eine intraperitoneale Challenge-Infektion mit 500 LD₅₀ des TBE-Virus. Die Beobachtungszeit betrug 3 Wochen. Die Ergebnisse des Antikörpernachweises und des Schutzversuchs sind in der Tabelle 5 zusammengefaßt. Wie daraus hervorgeht, konnte bei der DNA-Immunisierung ein Schutz gegen eine tödliche Infektion mit dem TBE-Virus nur mit jenem Plasmid erreicht werden, das die Gene für die vollständigen prM- und E-Proteine enthält. Dies ist wahrscheinlich dahingehend zu interpretieren, daß die Anwesenheit des prM/M-Proteins für den Zusammenbau von subviralen Partikeln, in welchen Protein E in immunogener Form vorliegt, notwendig ist.

**Tabelle 5 :**

| Ergebnisse des Mäuseschutzversuches | | |
|---|---|---|
| Immunogen | Schutz | Antikörper-positiv |
| Plasmide | | |
| PE-wt 60 µg/ml | 6^{a}/10^{b} | 1^{c}/10^{d} |
| PE-wt 300 µg/ml | 9/10 | 5/10 |
| E-wt 60 µg/ml | 0/10 | 0/10 |
| E-wt 300 µg/ml | 0/10 | 0/10 |
| PE-st 60 µg/ml | 0/10 | 0/10 |
| PE-st 300 µg/ml | 0/10 | 0/10 |
| E-st 60 µg/ml | 0/10 | 0/10 |
| E-st 300 µg/ml | 0/10 | 0/10 |
| CMV-β 300 µg/ml | 0/10 | 0/10 |

| Kontrollen | | |
|---|---|---|
| HCOH Virus 1 µg/ml | 4/10 | 4/10 |
| HCOH Virus 5 µg/ml | 10/10 | 10/10 |
| PBS | 0/10 | 0/10 |

| | | |
|---|---|---|
| ^{a} Anzahl der geschützten Mäuse | | |
| ^{b} Anzahl der untersuchten Mäuse | | |
| ^{c} Anzahl der ELISA-positiven Mäuse | | |
| ^{d} Anzahl der untersuchten Mäuse | | |

## Patentansprüche

1. Nackte Nukleinsäure-Vakzine zur Immunisierung gegen Flavi-Virus Infektionen, **dadurch gekennzeichnet, dass** sie eine Nukleinsäure, welche die für Protein E und Protein prM/M kodierende Nukleinsäuresequenz abgeleitet aus einem Flavi-Virus, in deren vollständiger, nativen Form enthält.

2. Vakzine nach Anspruch 1, **dadurch gekennzeichnet, dass** die für Protein E und prM/M kodierende Sequenz aus Tick-Borne-Enzephalitis-Virus (TBE-Virus) abgeleitet ist.

3. Vakzine nach Anspruch 1, **dadurch gekennzeichnet, dass** die für Protein E und prM/M kodierende Sequenz aus dem europäischen oder fernöstlichen Subtyp des TBE-Virus abgeleitet ist.

4. Vakzine nach einem der Ansprüche 1 bis 3, **dadurch gekennzeichnet, dass** die Nukleinsäure ein Plasmidvektor ist.

5. Vakzine nach Anspruch 4, **dadurch gekennzeichnet, dass** der Plasmidvektor von Plasmid CMVβ abgeleitet ist.

6. Verwendung einer nackten Nukleinsäure, die die für Protein E und Protein prM/M kodierenden Nukleinsäuresequenz abgeleitet aus einem Flavi-Virus in deren vollständiger, nativer Form enthält, zur Herstellung eines Arzneimittel.

7. Verwendung einer nackten Nukleinsäure, die die für Protein E und Protein prM/M kodierenden Nukleinsäuresequenz abgeleitet aus einem Flavi-Virus in deren vollständiger, nativer Form enthält, zur Herstellung einer Vakzine gegen Flavi-Virus-Infektionen.

8. Verwendung nach Anspruch 6 oder 7, **dadurch gekennzeichnet, dass** die Nukleinsäure die kodierenden Sequenzen für Protein E und Protein prM/M abgeleitet vom TBE-Virus enthält.

## Claims

1. A naked nucleic-acid vaccine for immunizing against flavivirus infections, **characterised in that** it comprises a nucleic acid which contains a protein E- and protein prM/M-encoding nucleic acid sequence derived from a flavivirus and provided in its complete, native form.

2. A vaccine according to claim 1, **characterised in that** the protein E- and prM/M-encoding sequence is derived from tick-borne encephalitis virus (TBE virus).

3. A vaccine according to claim 1, **characterised in that** the protein E- and prM/M-encoding sequence is derived from the European or Far East sub-type of the TBE virus.

4. A vaccine according to any one of claims 1 to 3, **characterised in that** the nucleic acid is a plasmid vector.

5. A vaccine according to claim 4, **characterised in that** the plasmid vector is derived from plasmid CMVβ.

6. The use of a naked nucleic acid comprising the protein E-and protein prM/M-encoding nucleic acid sequence derived from a flavivirus and provided in its complete, native form, for preparing a medicament.

7. The use of a naked nucleic acid comprising the protein E-and protein prM/M-encoding nucleic acid sequence derived from a flavivirus and provided in its complete, native form, for preparing a vaccine against flavivirus infections.

8. The use according to claim 6 or 7, **characterised in that** the nucleic acid comprises the protein E- and protein prM/M-encoding sequences derived from TBE virus.

## Revendications

1. Vaccin à acide nucléique nu, pour une immunisation contre les infections à flavi-virus, **caractérisé en ce qu'**il comporte un acide nucléique qui contient la séquence d'acides nucléiques codant pour la protéine E et la protéine prM/M, laquelle dérive d'un flavi-virus, sous sa forme native complète.

2. Vaccin selon la revendication 1, **caractérisé en ce que** la séquence codant pour la protéine E et/ou prM/M dérive du virus de l'encéphalite transmise par les tiques (virus TBE).

3. Vaccin selon la revendication 1, **caractérisé en ce que** la séquence codant pour la protéine E et prM/M dérive du sous-type européen ou extrêmeoriental du virus TBE.

4. Vaccin selon l'une des revendications 1 à 3, **caractérisé en ce que** l'acide nucléique est un vecteur plasmidique.

5. Vaccin selon la revendication 4, **caractérisé en ce que** le vecteur plasmidique dérive du plasmide CMVβ.

6. Utilisation d'un acide nucléique nu, qui contient la séquence d'acides nucléiques codant pour la protéine E et la protéine prM/M, qui dérive d'un flavi-virus sous sa forme native complète, pour préparer un médicament.

7. Utilisation d'un acide nucléique nu, qui contient la séquence d'acides nucléiques codant pour la protéine E et la protéine prM/M, qui dérive d'un flavi-virus sous sa forme native ou complète, pour préparer un vaccin contre les infections à flavi-virus.

8. Utilisation selon la revendication 6 ou 7, **caractérisée en ce que** l'acide nucléique contient les séquences codant pour la protéine E et la protéine prM/M, qui dérivent du virus TBE.
